# EUROPEAN PATENT APPLICATION

(11) **EP 3 553 080 A1**
(43) Date of publication of application: **16.10.2019**
(21) Application number: 18167111.6
(22) Date of filing: 12.04.2018
(51) Int. Cl.: C07K 14/705, C07K 14/47

(54) **PIEZO1-BASED FLUORESCENT REPORTER**

(71) Applicant: ETH Zürich, 8092 Zürich (CH)
(72) Inventor: PANTAZIS, Periklis, 4104 Oberwil (CH); YAGANOGLU, Sine, 8006 Zürich (CH)
(74) Representative: Schulz Junghans Patentanwälte PartGmbB

(57) **Abstract**

The invention relates to a polypeptide comprising a first polypeptide module comprising a calcium permeable transmembrane pore, the permeability of which is modulated by a stimulus, and a second polypeptide module comprising a luminescent moiety, a calcium binding moiety and a modulator peptide that can bind to the calcium binding moiety. The modulator has a higher affinity to the calcium binding moiety when it is bound to calcium, and luminescence alters upon binding of the modulator peptide sequence to the calcium binding moiety. The invention further relates to methods of using the polypeptide of the invention in detecting a stimulus, or in assaying test compounds.

SEQUENCE LISTING

<110>  ETH Z\237rich

<120>  Piezo1-based fluorescent reporter

<130>  eth171ep

<160>  27

<170>  PatentIn version 3.5

<210>  1
<211>  7563
<212>  DNA
<213>  Homo sapiens

<400>  1
atggagccgc acgtgctcgg cgcggtcctg tactggctgc tgctgccctg cgcgctgctg      60

gctgcctgcc tgctccgctt cagcggactc tcgctggtct acctgctctt cctgctgctg     120

ctgccctggt tccccggccc cacccgatgc ggcctccaag gtcacacagg ccgcctcctg     180

cgggcattgc tgggcctcag cctgctcttc ctggtggccc atctcgccct ccagatctgc     240

ctgcatactg tgccccgcct ggaccagctc ctgggaccca gctgcagccg ctgggagacc     300

ctctcgcgac acataggggt cacaaggctg gacctgaagg acatccccaa cgccatccgg     360

ctggtggccc ctgacctggg catcttggtg gtctcctctg tctgcctcgg catctgcggg     420

cgccttgcaa ggaacacccg gcagagccca catccacggg agctggatga tgatgagagg     480

gatgtggatg ccagcccgac ggcagggctg caggaagcag caacgctggc ccctacacgg     540

aggtcacggc tggccgctcg tttccgagtc acggcccact ggctgctggt ggcggctggg     600

cgggtcctgg ccgtaacact gcttgcactg gcaggcatcg cccacccctc ggccctctcc     660

agtgtctacc tgctgctctt cctggccctc tgcacctggt gggcctgcca ctttcccatc     720

agcactcggg gcttcagcag actctgcgcc gcggtggggt gcttcggcgc cggccatctc     780

atctgcctct actgctacca gatgcccttg gcacaggctc tgctcccgcc tgccggcatc     840

tgggctaggg tgctgggtct caaggacttc gtgggtccca ccaactgctc cagcccccac     900

gcgctggtcc tcaacaccgg cctggactgg cctgtgtatg ccagccccgg cgtcctcctg     960

ctgctgtgct acgccacggc ctctctgcgc aagctccgcg cgtaccgccc ctccggccag    1020

aggaaggagg cggcaaaggg gtatgaggct cgggagctgg agctagcaga gctggaccag    1080

tggccccagg aacgggagtc tgaccagcac gtggtgccca cagcacccga caccgaggct    1140

gataactgca tcgtgcacga gctgaccggc cagagctccc tcctgcggcg gcctgtgcgg    1200

cccaagcggg ctgagcccgg ggaggcgtct ccgctccaca gcctgggcca cctcatcatg    1260

gaccagagct atgtgtgcgc gctcattgcc atgatggtat ggagcatcac ctaccacagc    1320

tggctgacct tcgtactgct gctctgggcc tgcctcatct ggacggtgcg cagccgccac    1380

caactggcca tgctgtgctc gccctgcatc ctgctgtatg ggatgacgct gtgctgccta 1440

cgctacgtgt gggccatgga cctgcgccct gagctgccca ccaccctggg ccccgtcagc 1500

ctgcgccagc tggggctgga gcacacccgc tacccctgtc tggaccttgg tgccatgttg 1560

ctctacaccc tgaccttctg gctcctgctg cgccagtttg tgaaagagaa gctgctgaag 1620

tgggcagagt ctccagctgc gctgacggag gtcaccgtgg cagacacaga gcccacgcgg 1680

acgcagacgc tgttgcagag cctgggggag ctggtgaagg gcgtgtacgc caagtactgg 1740

atctatgtgt gtgctggcat gttcatcgtg gtcagcttcg ccggccgcct cgtggtctac 1800

aagattgtct acatgttcct cttcctgctc tgcctcaccc tcttccaggt ctactacagc 1860

ctgtggcgga agctgctcaa ggccttctgg tggctcgtgg tggcctacac catgctggtc 1920

ctcatcgccg tctacacctt ccagttccag gacttccctg cctactggcg caacctcact 1980

ggcttcaccg acgagcagct gggggacctg ggcctggagc agttcagcgt gtccgagctc 2040

ttctccagca tcctggtgcc cggcttcttc ctcctggcct gcatcctgca gctgcactac 2100

ttccacaggc ccttcatgca gctcaccgac atggagcacg tgtccctgcc tggcacgcgc 2160

ctcccgcgct gggctcacag gcaggatgca gtgagtggga ccccactgct gcgggaggag 2220

cagcaggagc atcagcagca gcagcaggag gaggaggagg aggaggagga ctccagggac 2280

gaggggctgg gcgtggccac tccccaccag gccacgcagg tgcctgaagg ggcagccaag 2340

tggggcctgg tggctgagcg gctgctggag ctggcagccg gcttctcgga cgtcctctca 2400

cgcgtgcagg tgttcctgcg gcggctgctg gagcttcacg ttttcaagct ggtggccctg 2460

tacaccgtct gggtggccct gaaggaggtg tcggtgatga acctgctgct ggtggtgctg 2520

tgggccttcg ccctgcccta cccacgcttc cggcccatgg cctcctgcct gtccaccgtg 2580

tggacctgcg tcatcatcgt gtgtaagatg ctgtaccagc tcaaggttgt caacccccag 2640

gagtattcca gcaactgcac cgagcccttc cccaacagca ccaacttgct gcccacggag 2700

atcagccagt ccctgctgta ccgggggccc gtggaccctg ccaactggtt tggggtgcgg 2760

aaagggttcc ccaacctggg ctacatccag aaccacctgc aagtgctgct gctgctggta 2820

ttcgaggcca tcgtgtaccg gcgccaggag cactaccgcc ggcagcacca gctggccccg 2880

ctgcctgccc aggccgtgtt tgccagcggc acccgccagc agctggacca ggatctgctc 2940

ggctgcctca agtacttcat caacttcttc ttctacaaat tcgggctgga gatctgcttc 3000

ctgatggccg tgaacgtgat cgggcagcgc atgaactttc tggtgaccct gcacggttgc 3060

tggctggtgg ccatcctcac ccgcaggcac cgccaggcca ttgcccgcct ctggcccaac 3120

tactgcctct tcctggcgct gttcctgctg taccagtacc tgctgtgcct ggggatgccc 3180

ccggccctgt gcattgatta tccctggcgc tggagccggg ccgtccccat gaactccgca 3240

ctcatcaagt ggctgtacct gcctgatttc ttccgggccc ccaactccac caacctcatc 3300

agcgactttc tcctgctgct gtgcgcctcc cagcagtggc aggtgttctc agctgagcgc 3360

acagaggagt ggcagcgcat ggctggcgtc aacaccgacc gcctggagcc gctgcggggg 3420

gagcccaacc ccgtgcccaa ctttatccac tgcaggtcct accttgacat gctgaaggtg 3480

gccgtcttcc gatacctgtt ctggctggtg ctggtggtgg tgtttgtcac gggggccacc 3540

cgcatcagca tcttcgggct gggctacctg ctggcctgct tctacctgct gctcttcggc 3600

acggccctgc tgcagaggga cacacgggcc cgcctcgtgc tgtgggactg cctcattctg 3660

tacaacgtca ccgtcatcat ctccaagaac atgctgtcgc tcctggcctg cgtcttcgtg 3720

gagcagatgc agaccggctt ctgctgggtc atccagctct tcagccttgt atgcaccgtc 3780

aagggctact atgaccccaa ggagatgatg gacagagacc aggactgcct gctgcctgtg 3840

gaggaggctg gcatcatctg ggacagcgtc tgcttcttct tcctgctgct gcagcgccgc 3900

gtcttcctta gccattacta cctgcacgtc agggccgacc tccaggccac cgccctgcta 3960

gcctccaggg gcttcgccct ctacaacgct gccaacctca agagcattga cttccaccgc 4020

aggatagagg agaagtccct ggcccagctg aaaagacaga tggagcgtat ccgtgccaag 4080

caggagaagc acaggcaggg ccgggtggac cgcagtcgcc cccaggacac cctgggcccc 4140

aaggaccccg gcctggagcc agggcccgac agtccagggg gctcctcccc gccacggagg 4200

cagtggtggc ggccctggct ggaccacgcc acagtcatcc actccgggga ctacttcctg 4260

tttgagtccg acagtgagga agaggaggag gctgttcctg aagacccgag gccgtcggca 4320

cagagtgcct tccagctggc gtaccaggca tgggtgacca acgcccaggc ggtgctgagg 4380

cggcggcagc aggagcagga gcaggcaagg caggaacagg caggacagct acccacagga 4440

ggtggtccca gccaggaggt ggagccagca gagggccccg aggaggcagc ggcaggccgg 4500

agccatgtgg tgcagagggt gctgagcacg gcgcagttcc tgtggatgct ggggcaggcg 4560

ctagtggatg agctgacacg ctggctgcag gagttcaccc ggcaccacgg caccatgagc 4620

gacgtgctgc gggcagagcg ctacctcctc acacaggagc tcctgcaggg cggcgaagtg 4680

cacaggggcg tgctggatca gctgtacaca agccaggccg aggccacgct gccaggcccc 4740

accgaggccc ccaatgcccc aagcaccgtg tccagtgggc tgggcgcgga ggagccactc 4800

agcagcatga cagacgacat gggcagcccc ctgagcaccg gctaccacac gcgcagtggc 4860

agtgaggagg cagtcaccga ccccggggag cgtgaggctg gtgcctctct gtaccaggga 4920

ctgatgcgga cggccagcga gctgctcctg gacaggcgcc tgcgcatccc agagctggag 4980

gaggcagagc tgtttgcgga ggggcagggc cgggcgctgc ggctgctgcg ggccgtgtac 5040

cagtgtgtgg ccgcccactc ggagctgctc tgctacttca tcatcatcct caaccacatg 5100

gtcacggcct ccgccggctc gctggtgctg cccgtgctcg tcttcctgtg ggccatgctg 5160

tcgatcccga ggcccagcaa gcgcttctgg atgacggcca tcgtcttcac cgagatcgcg 5220

gtggtcgtca agtacctgtt ccagtttggg ttcttcccct ggaacagcca cgtggtgctg 5280

cggcgctacg agaacaagcc ctacttcccg ccccgcatcc tgggcctgga gaagactgac 5340

ggctacatca agtacgacct ggtgcagctc atggcccttt tcttccaccg ctcccagctg 5400

ctgtgctatg gcctctggga ccatgaggag gactcaccat ccaaggagca tgacaagagc 5460

ggcgaggagg agcagggagc cgaggagggg ccaggggtgc ctgcggccac caccgaagac 5520

cacattcagg tggaagcgag ggtcggaccc acggacggga ccccagaacc ccaagtggag 5580

ctcaggcccc gtgatacgag gcgcatcagt ctacgtttta gaagaaggaa gaaggagggc 5640

ccagcacgga aaggagcggc agccatcgaa gctgaggaca gggaggaaga agagggggag 5700

gaagagaaag aggcccccac ggggagagag aagaggccaa gccgctctgg aggaagagta 5760

agggcggccg ggcggcggct gcagggcttc tgcctgtccc tggcccaggg cacatatcgg 5820

ccgctacggc gcttcttcca cgacatcctg cacaccaagt accgcgcagc caccgacgtc 5880

tatgccctca tgttcctggc tgatgttgtc gacttcatca tcatcatttt tggcttctgg 5940

gcctttggga agcactcggc ggccacagac atcacgtcct ccctatcaga cgaccaggta 6000

cccgaggctt tcctggtcat gctgctgatc cagttcagta ccatggtggt tgaccgcgcc 6060

ctctacctgc gcaagaccgt gctgggcaag ctggccttcc aggtggcgct ggtgctggcc 6120

atccacctat ggatgttctt catcctgccc gccgtcactg agaggatgtt caaccagaat 6180

gtggtggccc agctctggta cttcgtgaag tgcatctact tcgccctgtc cgcctaccag 6240

atccgctgcg gctaccccac ccgcatcctc ggcaacttcc tcaccaagaa gtacaatcat 6300

ctcaacctct tcctcttcca ggggttccgg ctggtgccgt tcctggtgga gctgcgggca 6360

gtgatggact gggtgtggac ggacaccacg ctgtccctgt ccagctggat gtgtgtggag 6420

gacatctatg ccaacatctt catcatcaaa tgcagccgag agacagagaa gaaatacccg 6480

cagcccaaag ggcagaagaa gaagaagatc gtcaagtacg gcatgggtgg cctcatcatc 6540

ctcttcctca tcgccatcat ctggttcccg ctgctcttca tgtcgctggt gcgctccgtg 6600

gttggggttg tcaaccagcc catcgatgtc accgtcaccc tcaagctggg cggctatgag 6660

ccgctgttca ccatgagcgc ccagcagccg tccatcatcc ccttcacggc ccaggcctat 6720

gaggagctgt cccggcagtt tgacccccag ccgctggcca tgcagttcat cagccagtac 6780

agccctgagg acatcgtcac ggcgcagatt gagggcagct ccggggcgct gtggcgcatc 6840

agtcccccca gccgtgccca gatgaagcgg gagctctaca acggcacggc cgacatcacc 6900

ctgcgcttca cctggaactt ccagagggac ctggcgaagg gaggcactgt ggagtatgcc 6960

aacgagaagc acatgctggc cctggccccc aacagcactg cacggcggca gctggccagc 7020

ctgctcgagg gcacctcgga ccagtctgtg gtcatcccca atctcttccc caagtacatc 7080

cgtgccccca acgggcccga agccaaccct gtgaagcagc tgcagcccaa tgaggaggcc 7140

gactacctcg gcgtgcgtat ccagctgcgg agggagcagg gtgcgggggc caccggcttc 7200

ctcgaatggt gggtcatcga gctgcaggag tgccggaccg actgcaacct gctgcccatg 7260

gtcattttca gtgacaaggt cagcccaccg agcctcggct tcctggctgg ctacggcatc 7320

atggggctgt acgtgtccat cgtgctggtc atcggcaagt tcgtgcgcgg attcttcagc 7380

gagatctcgc actccattat gttcgaggag ctgccgtgcg tggaccgcat cctcaagctc 7440

tgccaggaca tcttcctggt gcgggagact cgggagctgg agctggagga ggagttgtac 7500

gccaagctca tcttcctcta ccgctcaccg gagaccatga tcaagtggac tcgtgagaag 7560

gag 7563

<210> 2
<211> 714
<212> DNA
<213> Artificial Sequence

<220>
<223> artificial sequence

<400> 2
gccgacaagc agaagaacgg catcaaggcg aacttccaca tccgccacaa catcgaggac 60

ggcggcgtgc agctcgccta ccactaccag cagaacaccc ccatcggcga cggccccgtg 120

ctgctgcccg acaaccacta cctgagcgtg cagtccaaac tttcgaaaga ccccaacgag 180

aagcgcgatc acatggtcct gctggagttc gtgaccgccg ccgggatcac tctcggcatg 240

gacgagctgt acaagggcgg taccggaggg agcatggtga gcaagggcga ggagctgttc 300

accggggtgg tgcccatcct ggtcgagctg gacggcgacg taaacggcca caagttcagc 360

gtgtccggcg agggtgaggg cgatgccacc tacggcaagc tgaccctgaa gttcatctgc 420

accaccggca agctgcccgt gccctggccc accctcgtga ccaccctgac ctacggcgtg 480

cagtgcttca gccgctaccc cgaccacatg aagcagcacg acttcttcaa gtccgccatg 540

cccgaaggct acatccagga gcgcaccatc ttcttcaagg acgacggcaa ctacaagacc 600

cgcgccgagg tgaagttcga gggcgacacc ctggtgaacc gcatcgagct gaagggcatc 660

gacttcaagg aggacggcaa catcctgggg cacaagctgg agtacaacct gccg 714

<210> 3
<211> 444
<212> DNA
<213> Artificial Sequence

<220>
<223> artificial sequence

<400>  3
gaccaactga ctgaagagca gatcgcagaa tttaaagagg ctttctccct atttgacaag      60

gacggggatg ggacaataac aaccaaggag ctggggacgg tgatgcggtc tctggggcag     120

aaccccacag aagcagagct gcaggacatg atcaatgaag tagatgccga cggtgacggc     180

acaatcgact tccctgagtt cctgacaatg atggcaagaa aaatgaaata cagggacacg     240

gaagaagaaa ttagagaagc gttcggtgtg tttgataagg atggcaatgg ctacatcagt     300

gcagcagagc ttcgccacgt gatgacaaac cttggagaga agttaacaga tgaagaggtt     360

gatgaaatga tcagggaagc agccatcgat ggggatggtc aggtaaacta cgaagagttt     420

gtacaaatga tgacagcgaa gtaa                                            444


<210>  4
<211>  21
<212>  PRT
<213>  Artificial Sequence

<220>
<223>  RS20 peptide sequence

<400>  4

Ser Ser Arg Arg Lys Tyr Asn Lys Thr Gly His Ala Val Arg Ala Ile
1               5                   10                  15


Gly Arg Leu Ser Ser
            20


<210>  5
<211>  22
<212>  PRT
<213>  Artificial Sequence

<220>
<223>  modulator  peptide sequence

<400>  5

Ala Lys Ser Lys Trp Lys Gln Ala Phe Asn Ala Thr Ala Val Val Arg
1               5                   10                  15


His Met Arg Lys Leu Gln
            20


<210>  6
<211>  23
<212>  PRT
<213>  Artificial Sequence

<220>
<223>  modulator  peptide sequence

<400>  6

Lys Arg Arg Ala Ile Gly Phe Lys Lys Leu Ala Glu Ala Val Lys Phe
1               5                   10                  15

Ser Ala Lys Leu Met Gly Gln
            20

<210>  7
<211>  20
<212>  PRT
<213>  Artificial Sequence

<220>
<223>  modulator  peptide sequence

<400>  7

Gln Thr Glu Lys Met Trp Gln Arg Leu Lys Gly Ile Leu Arg Cys Leu
1               5                   10                  15

Val Lys Gln Leu
            20

<210>  8
<211>  16
<212>  PRT
<213>  Artificial Sequence

<220>
<223>  modulator  peptide sequence

<400>  8

Gly Val Asn Ile Lys Ser Met Trp Glu Lys Gly Asn Val Phe Ser Ser
1               5                   10                  15

<210>  9
<211>  22
<212>  PRT
<213>  Artificial Sequence

<220>
<223>  modulator  peptide sequence

<400>  9

Asn Lys Glu Thr Ala Gly Leu Lys Val Gly Val Ser Ser Arg Ile Asn
1               5                   10                  15

Glu Trp Leu Thr Lys Thr
            20

<210>  10
<211>  26
<212>  PRT
<213>  Artificial Sequence

<220>
<223>  modulator  peptide sequence

<400>  10

Gln Gln Arg Lys Arg Lys Ile Trp Ser Ile Leu Ala Pro Leu Gly Thr
1               5                   10                  15

Thr Leu Val Lys Leu Val Ala Gly Ile Gly
            20                  25

<210>  11
<211>  13
<212>  PRT
<213>  Artificial Sequence

<220>
<223>  modulator  peptide sequence

<400>  11

Gln Gln Arg Lys Arg Lys Ile Trp Ser Ile Leu Ala Pro
1               5                   10

<210>  12
<211>  20
<212>  PRT
<213>  Artificial Sequence

<220>
<223>  modulator  peptide sequence

<400>  12

Ala Arg Arg Lys Trp Gln Lys Thr Gly His Ala Val Arg Ala Ile Gly
1               5                   10                  15

Arg Leu Ser Ser
            20

<210>  13
<211>  1353
<212>  DNA
<213>  Artificial Sequence

<220>
<223>  fusion proetin cpGFP RS20 peptide Calmodulin

<400>  13
atgggttctc atcatcatca tcatcatggt atggctagca tgactggtgg acagcaaatg      60

ggtcgggatc tgtacgacga tgacgataag gatctcgcca ccatggtcga ctcatcacgt     120

cgtaagtaca ataagacagg tcacgcagtc agagctatag gtcggctgag ctcactcgag     180

aacgtctata tcaaggccga caagcagaag aacggcatca aggcgaactt ccacatccgc     240

cacaacatcg aggacggcgg cgtgcagctc gcctaccact accagcagaa cacccccatc 300

ggcgacggcc ccgtgctgct gcccgacaac cactacctga gcgtgcagtc caaactttcg 360

aaagacccca acgagaagcg cgatcacatg gtcctgctgg agttcgtgac cgccgccggg 420

atcactctcg gcatggacga gctgtacaag ggcggtaccg gagggagcat ggtgagcaag 480

ggcgaggagc tgttcaccgg ggtggtgccc atcctggtcg agctggacgg cgacgtaaac 540

ggccacaagt tcagcgtgtc cggcgagggt gagggcgatg ccacctacgg caagctgacc 600

ctgaagttca tctgcaccac cggcaagctg cccgtgccct ggcccaccct cgtgaccacc 660

ctgacctacg gcgtgcagtg cttcagccgc taccccgacc acatgaagca gcacgacttc 720

ttcaagtccg ccatgcccga aggctacatc caggagcgca ccatcttctt caaggacgac 780

ggcaactaca agacccgcgc cgaggtgaag ttcgagggcg acaccctggt gaaccgcatc 840

gagctgaagg gcatcgactt caaggaggac ggcaacatcc tggggcacaa gctggagtac 900

aacctgccgg accaactgac tgaagagcag atcgcagaat ttaaagaggc tttctcccta 960

tttgacaagg acggggatgg gacaataaca accaaggagc tggggacggt gatgcggtct 1020

ctggggcaga accccacaga agcagagctg caggacatga tcaatgaagt agatgccgac 1080

ggtgacggca caatcgactt ccctgagttc ctgacaatga tggcaagaaa aatgaaatac 1140

agggacacgg aagaagaaat tagagaagcg ttcggtgtgt ttgataagga tggcaatggc 1200

tacatcagtg cagcagagct tcgccacgtg atgacaaacc ttggagagaa gttaacagat 1260

gaagaggttg atgaaatgat cagggaagca gccatcgatg gggatggtca ggtaaactac 1320

gaagagtttg tacaaatgat gacagcgaag taa 1353

<210> 14
<211> 8933
<212> DNA
<213> Artificial Sequence

<220>
<223> Fusion protein human Piezo1 cpGFP RS20 peptide Calmodulin

<400> 14
atggagccgc acgtgctcgg cgcggtcctg tactggctgc tgctgccctg cgcgctgctg 60

gctgcctgcc tgctccgctt cagcggactc tcgctggtct acctgctctt cctgctgctg 120

ctgccctggt tccccggccc cacccgatgc ggcctccaag gtcacacagg ccgcctcctg 180

cgggcattgc tgggcctcag cctgctcttc ctggtggccc atctcgccct ccagatctgc 240

ctgcatactg tgccccgcct ggaccagctc ctgggaccca gctgcagccg ctgggagacc 300

ctctcgcgac acataggggt cacaaggctg gacctgaagg acatccccaa cgccatccgg 360

ctggtggccc ctgacctggg catcttggtg gtctcctctg tctgcctcgg catctgcggg 420

cgccttgcaa ggaacacccg gcagagccca catccacggg agctggatga tgatgagagg 480

gatgtggatg ccagcccgac ggcagggctg caggaagcag caacgctggc ccctacacgg 540

aggtcacggc tggccgctcg tttccgagtc acggcccact ggctgctggt ggcggctggg 600

cgggtcctgg ccgtaacact gcttgcactg gcaggcatcg cccacccctc ggccctctcc 660

agtgtctacc tgctgctctt cctggccctc tgcacctggt gggcctgcca ctttcccatc 720

agcactcggg gcttcagcag actctgcgcc gcggtggggt gcttcggcgc cggccatctc 780

atctgcctct actgctacca gatgcccttg gcacaggctc tgctcccgcc tgccggcatc 840

tgggctaggg tgctgggtct caaggacttc gtgggtccca ccaactgctc cagcccccac 900

gcgctggtcc tcaacaccgg cctggactgg cctgtgtatg ccagccccgg cgtcctcctg 960

ctgctgtgct acgccacggc ctctctgcgc aagctccgcg cgtaccgccc ctccggccag 1020

aggaaggagg cggcaaaggg gtatgaggct cgggagctgg agctagcaga gctggaccag 1080

tggccccagg aacgggagtc tgaccagcac gtggtgccca cagcacccga caccgaggct 1140

gataactgca tcgtgcacga gctgaccggc cagagctccc tcctgcggcg gcctgtgcgg 1200

cccaagcggg ctgagcccgg ggaggcgtct ccgctccaca gcctgggcca cctcatcatg 1260

gaccagagct atgtgtgcgc gctcattgcc atgatggtat ggagcatcac ctaccacagc 1320

tggctgacct tcgtactgct gctctgggcc tgcctcatct ggacggtgcg cagccgccac 1380

caactggcca tgctgtgctc gccctgcatc ctgctgtatg ggatgacgct gtgctgccta 1440

cgctacgtgt gggccatgga cctgcgccct gagctgccca ccaccctggg ccccgtcagc 1500

ctgcgccagc tggggctgga gcacacccgc tacccctgtc tggaccttgg tgccatgttg 1560

ctctacaccc tgaccttctg gctcctgctg cgccagtttg tgaaagagaa gctgctgaag 1620

tgggcagagt ctccagctgc gctgacggag gtcaccgtgg cagacacaga gcccacgcgg 1680

acgcagacgc tgttgcagag cctgggggag ctggtgaagg gcgtgtacgc caagtactgg 1740

atctatgtgt gtgctggcat gttcatcgtg gtcagcttcg ccggccgcct cgtggtctac 1800

aagattgtct acatgttcct cttcctgctc tgcctcaccc tcttccaggt ctactacagc 1860

ctgtggcgga agctgctcaa ggccttctgg tggctcgtgg tggcctacac catgctggtc 1920

ctcatcgccg tctacacctt ccagttccag gacttccctg cctactggcg caacctcact 1980

ggcttcaccg acgagcagct gggggacctg ggcctggagc agttcagcgt gtccgagctc 2040

ttctccagca tcctggtgcc cggcttcttc ctcctggcct gcatcctgca gctgcactac 2100

ttccacaggc ccttcatgca gctcaccgac atggagcacg tgtccctgcc tggcacgcgc 2160

ctcccgcgct gggctcacag gcaggatgca gtgagtggga ccccactgct gcgggaggag 2220

cagcaggagc atcagcagca gcagcaggag gaggaggagg aggaggagga ctccagggac 2280

gaggggctgg gcgtggccac tccccaccag gccacgcagg tgcctgaagg ggcagccaag 2340

tggggcctgg tggctgagcg gctgctggag ctggcagccg gcttctcgga cgtcctctca 2400
cgcgtgcagg tgttcctgcg gcggctgctg gagcttcacg ttttcaagct ggtggccctg 2460
tacaccgtct gggtggccct gaaggaggtg tcggtgatga acctgctgct ggtggtgctg 2520
tgggccttcg ccctgcccta cccacgcttc cggcccatgg cctcctgcct gtccaccgtg 2580
tggacctgcg tcatcatcgt gtgtaagatg ctgtaccagc tcaaggttgt caacccccag 2640
gagtattcca gcaactgcac cgagcccttc cccaacagca ccaacttgct gcccacggag 2700
atcagccagt ccctgctgta ccgggggccc gtggaccctg ccaactggtt tggggtgcgg 2760
aaagggttcc ccaacctggg ctacatccag aaccacctgc aagtgctgct gctgctggta 2820
ttcgaggcca tcgtgtaccg gcgccaggag cactaccgcc ggcagcacca gctggccccg 2880
ctgcctgccc aggccgtgtt tgccagcggc acccgccagc agctggacca ggatctgctc 2940
ggctgcctca agtacttcat caacttcttc ttctacaaat tcgggctgga gatctgcttc 3000
ctgatggccg tgaacgtgat cgggcagcgc atgaactttc tggtgaccct gcacggttgc 3060
tggctggtgg ccatcctcac ccgcaggcac cgccaggcca ttgcccgcct ctggcccaac 3120
tactgcctct tcctggcgct gttcctgctg taccagtacc tgctgtgcct ggggatgccc 3180
ccggccctgt gcattgatta tccctggcgc tggagccggg ccgtccccat gaactccgca 3240
ctcatcaagt ggctgtacct gcctgatttc ttccgggccc ccaactccac caacctcatc 3300
agcgactttc tcctgctgct gtgcgcctcc cagcagtggc aggtgttctc agctgagcgc 3360
acagaggagt ggcagcgcat ggctggcgtc aacaccgacc gcctggagcc gctgcggggg 3420
gagcccaacc ccgtgcccaa ctttatccac tgcaggtcct accttgacat gctgaaggtg 3480
gccgtcttcc gatacctgtt ctggctggtg ctggtggtgg tgtttgtcac gggggccacc 3540
cgcatcagca tcttcgggct gggctacctg ctggcctgct tctacctgct gctcttcggc 3600
acggccctgc tgcagaggga cacacgggcc cgcctcgtgc tgtgggactg cctcattctg 3660
tacaacgtca ccgtcatcat ctccaagaac atgctgtcgc tcctggcctg cgtcttcgtg 3720
gagcagatgc agaccggctt ctgctgggtc atccagctct tcagccttgt atgcaccgtc 3780
aagggctact atgaccccaa ggagatgatg gacagagacc aggactgcct gctgcctgtg 3840
gaggaggctg gcatcatctg ggacagcgtc tgcttcttct tcctgctgct gcagcgccgc 3900
gtcttcctta gccattacta cctgcacgtc agggccgacc tccaggccac cgccctgcta 3960
gcctccaggg gcttcgccct ctacaacgct gccaacctca agagcattga cttccaccgc 4020
aggatagagg agaagtccct ggcccagctg aaaagacaga tggagcgtat ccgtgccaag 4080
caggagaagc acaggcaggg ccgggtggac cgcagtcgcc cccaggacac cctgggcccc 4140
aaggaccccg gcctggagcc agggcccgac agtccagggg gctcctcccc gccacggagg 4200
cagtggtggc ggccctggct ggaccacgcc acagtcatcc actccgggga ctacttcctg 4260

tttgagtccg acagtgagga agaggaggag gctgttcctg aagacccgag gccgtcggca 4320

cagagtgcct tccagctggc gtaccaggca tgggtgacca acgcccaggc ggtgctgagg 4380

cggcggcagc aggagcagga gcaggcaagg caggaacagg caggacagct acccacagga 4440

ggtggtccca gccaggaggt ggagccagca gagggccccg aggaggcagc ggcaggccgg 4500

agccatgtgg tgcagagggt gctgagcacg gcgcagttcc tgtggatgct ggggcaggcg 4560

ctagtggatg agctgacacg ctggctgcag gagttcaccc ggcaccacgg caccatgagc 4620

gacgtgctgc gggcagagcg ctacctcctc acacaggagc tcctgcaggg cggcgaagtg 4680

cacagggcgg tgctggatca gctgtacaca agccaggccg aggccacgct gccaggcccc 4740

accgaggccc ccaatgcccc aagcaccgtg tccagtgggc tgggcgcgga ggagccactc 4800

agcagcatga cagacgacat gggcagcccc ctgagcaccg gctaccacac gcgcagtggc 4860

agtgaggagg cagtcaccga ccccggggag cgtgaggctg gtgcctctct gtaccaggga 4920

ctgatgcgga cggccagcga gctgctcctg gacaggcgcc tgcgcatccc agagctggag 4980

gaggcagagc tgtttgcgga ggggcagggc cgggcgctgc ggctgctgcg ggccgtgtac 5040

cagtgtgtgg ccgcccactc ggagctgctc tgctacttca tcatcatcct caaccacatg 5100

gtcacggcct ccgccggctc gctggtgctg cccgtgctcg tcttcctgtg ggccatgctg 5160

tcgatcccga ggcccagcaa gcgcttctgg atgacggcca tcgtcttcac cgagatcgcg 5220

gtggtcgtca agtacctgtt ccagtttggg ttcttcccct ggaacagcca cgtggtgctg 5280

cggcgctacg agaacaagcc ctacttcccg ccccgcatcc tgggcctgga gaagactgac 5340

ggctacatca agtacgacct ggtgcagctc atggcccttt tcttccaccg ctcccagctg 5400

ctgtgctatg gcctctggga ccatgaggag gactcaccat ccaaggagca tgacaagagc 5460

ggcgaggagg agcagggagc cgaggagggg ccaggggtgc ctgcggccac caccgaagac 5520

cacattcagg tggaagcgag ggtcggaccc acggacggga ccccagaacc ccaagtggag 5580

ctcaggcccc gtgatacgag gcgcatcagt ctacgtttta gaagaaggaa gaaggagggc 5640

ccagcacgga aaggagcggc agccatcgaa gctgaggaca gggaggaaga agagggggag 5700

gaagagaaag aggcccccac ggggagagag aagaggccaa gccgctctgg aggaagagta 5760

agggcggccg ggcggcggct gcagggcttc tgcctgtccc tggcccaggg cacatatcgg 5820

ccgctacggc gcttcttcca cgacatcctg cacaccaagt accgcgcagc caccgacgtc 5880

tatgccctca tgttcctggc tgatgttgtc gacttcatca tcatcatttt tggcttctgg 5940

gcctttggga agcactcggc ggccacagac atcacgtcct ccctatcaga cgaccaggta 6000

cccgaggctt tcctggtcat gctgctgatc cagttcagta ccatggtggt tgaccgcgcc 6060

ctctacctgc gcaagaccgt gctgggcaag ctggccttcc aggtggcgct ggtgctggcc 6120

atccacctat ggatgttctt catcctgccc gccgtcactg agaggatgtt caaccagaat 6180

gtggtggccc agctctggta cttcgtgaag tgcatctact tcgccctgtc cgcctaccag 6240

atccgctgcg gctaccccac ccgcatcctc ggcaacttcc tcaccaagaa gtacaatcat 6300

ctcaacctct tcctcttcca ggggttccgg ctggtgccgt tcctggtgga gctgcgggca 6360

gtgatggact gggtgtggac ggacaccacg ctgtccctgt ccagctggat gtgtgtggag 6420

gacatctatg ccaacatctt catcatcaaa tgcagccgag agacagagaa gaaataccgg 6480

cagcccaaag ggcagaagaa gaagaagatc gtcaagtacg gcatgggtgg cctcatcatc 6540

ctcttcctca tcgccatcat ctggttcccg ctgctcttca tgtcgctggt gcgctccgtg 6600

gttggggttg tcaaccagcc catcgatgtc accgtcaccc tcaagctggg cggctatgag 6660

ccgctgttca ccatgagcgc ccagcagccg tccatcatcc ccttcacggc ccaggcctat 6720

gaggagctgt cccggcagtt tgacccccag ccgctggcca tgcagttcat cagccagtac 6780

agccctgagg acatcgtcac ggcgcagatt gagggcagct ccggggcgct gtggcgcatc 6840

agtcccccca gccgtgccca gatgaagcgg gagctctaca acggcacggc cgacatcacc 6900

ctgcgcttca cctggaactt ccagagggac ctggcgaagg gaggcactgt ggagtatgcc 6960

aacgagaagc acatgctggc cctggccccc aacagcactg cacggcggca gctggccagc 7020

ctgctcgagg gcacctcgga ccagtctgtg gtcatcccca atctcttccc caagtacatc 7080

cgtgccccca acgggcccga agccaaccct gtgaagcagc tgcagcccaa tgaggaggcc 7140

gactacctcg gcgtgcgtat ccagctgcgg agggagcagg gtgcgggggc caccggcttc 7200

ctcgaatggt gggtcatcga gctgcaggag tgccggaccg actgcaacct gctgcccatg 7260

gtcattttca gtgacaaggt cagcccaccg agcctcggct tcctggctgg ctacggcatc 7320

atggggctgt acgtgtccat cgtgctggtc atcggcaagt tcgtgcgcgg attcttcagc 7380

gagatctcgc actccattat gttcgaggag ctgccgtgcg tggaccgcat cctcaagctc 7440

tgccaggaca tcttcctggt gcgggagact cgggagctgg agctggagga ggagttgtac 7500

gccaagctca tcttcctcta ccgctcaccg gagaccatga tcaagtggac tcgtgagaag 7560

gagaccggtg gaggatccgg catgggttct catcatcatc atcatcatgg tatggctagc 7620

atgactggtg gacagcaaat gggtcgggat ctgtacgacg atgacgataa ggatctcgcc 7680

accatggtcg actcatcacg tcgtaagtac aataagacag gtcacgcagt cagagctata 7740

ggtcggctga gctcactcga gaacgtctat atcaaggccg acaagcagaa gaacggcatc 7800

aaggcgaact tccacatccg ccacaacatc gaggacggcg gcgtgcagct cgccaccact 7860

accagcagaa cacccccatc ggcgacggcc ccgtgctgct gcccgacaac cactacctga 7920

gcgtgcagtc caaactttcg aaagacccca acgagaagcg cgatcacatg gtcctgctgg 7980

agttcgtgac cgccgccggg atcactctcg gcatggacga gctgtacaag ggcggtaccg 8040

gagggagcat ggtgagcaag ggcgaggagc tgttcaccgg ggtggtgccc atcctggtcg 8100

agctggacgg cgacgtaaac ggccacaagt tcagcgtgtc cggcgagggt gagggcgatg 8160

ccacctacgg caagctgacc ctgaagttca tctgcaccac cggcaagctg cccgtgccct 8220

ggcccaccct cgtgaccacc ctgacctacg gcgtgcagtg cttcagccgc taccccgacc 8280

acatgaagca gcacgacttc ttcaagtccg ccatgcccga aggctacatc caggagcgca 8340

ccatcttctt caaggacgac ggcaactaca agacccgcgc cgaggtgaag ttcgagggcg 8400

acaccctggt gaaccgcatc gagctgaagg gcatcgactt caaggaggac ggcaacatcc 8460

tggggcacaa gctggagtac aacctgccgg accaactgac tgaagagcag atcgcagaat 8520

ttaaagaggc tttctcccta tttgacaagg acggggatgg gacaataaca accaaggagc 8580

tggggacggt gatgcggtct ctggggcaga accccacaga agcagagctg caggacatga 8640

tcaatgaagt agatgccgac ggtgacggca caatcgactt ccctgagttc ctgacaatga 8700

tggcaagaaa aatgaaatac agggacacgg aagaagaaat tagagaagcg ttcggtgtgt 8760

ttgataagga tggcaatggc tacatcagtg cagcagagct tcgccacgtg atgacaaacc 8820

ttggagagaa gttaacagat gaagaggttg atgaaatgat cagggaagca gccatcgatg 8880

gggatggtca ggtaaactac gaagagtttg tacaaatgat gacagcgaag taa 8933

<210> 15
<211> 909
<212> DNA
<213> Artificial Sequence

<220>
<223> Fusion Protein RS20 peptide cpGFP Calmodulin

<400> 15
atgggttctc atcatcatca tcatcatggt atggctagca tgactggtgg acagcaaatg 60

ggtcgggatc tgtacgacga tgacgataag gatctcgcca ccatggtcga ctcatcacgt 120

cgtaagtaca ataagacagg tcacgcagtc agagctatag gtcggctgag ctcactcgag 180

aacgtctata tcaaggccga caagcagaag aacggcatca aggcgaactt ccacatccgc 240

cacaacatcg aggacggcgg cgtgcagctc gcctaccact accagcagaa cacccccatc 300

ggcgacggcc ccgtgctgct gcccgacaac cactacctga gcgtgcagtc caaactttcg 360

aaagacccca acgagaagcg cgatcacatg gtcctgctgg agttcgtgac cgccgccggg 420

atcactctcg gcatggacga gctgtacaag ggcggtaccg gagggagcat ggtgagcaag 480

ggcgaggagc tgttcaccgg ggtggtgccc atcctggtcg agctggacgg cgacgtaaac 540

ggccacaagt tcagcgtgtc cggcgagggt gagggcgatg ccacctacgg caagctgacc 600

ctgaagttca tctgcaccac cggcaagctg cccgtgccct ggcccaccct cgtgaccacc 660

ctgacctacg gcgtgcagtg cttcagccgc taccccgacc acatgaagca gcacgacttc      720

ttcaagtccg ccatgcccga aggctacatc caggagcgca ccatcttctt caaggacgac      780

ggcaactaca agacccgcgc cgaggtgaag ttcgagggcg acaccctggt gaaccgcatc      840

gagctgaagg gcatcgactt caaggaggac ggcaacatcc tggggcacaa gctggagtac      900

aacctgccg                                                              909


<210>  16
<211>  32
<212>  DNA
<213>  artificial

<220>
<223>  PCR primer

<400>  16
ccaaccggta tgggttctca tcatcatcat ca                                    32


<210>  17
<211>  44
<212>  DNA
<213>  Artificial Sequence

<220>
<223>  PCR primer

<400>  17
ccaaccggtg gaggatccgg catgggttct catcatcatc atca                       44


<210>  18
<211>  56
<212>  DNA
<213>  Artificial Sequence

<220>
<223>  PCR primer

<400>  18
ccaaccggtg gatcaggagg tggatcagga ggtatgggtt ctcatcatca tcatca          56


<210>  19
<211>  52
<212>  DNA
<213>  Artificial Sequence

<220>
<223>  PCR primer

<400>  19
ttttcctgcg gccgcttttt tccttcctta cttcgctgtc atcatttgta ca              52


<210>  20
<211>  25
<212>  DNA
<213>  Artificial Sequence

<220>
<223>  PCR primer

<400>  20
gcagcctagg tattaatcaa ttagt                                          25


<210>  21
<211>  28
<212>  DNA
<213>  Artificial Sequence

<220>
<223>  PCR primer

<400>  21
ccaaagctta tcgcatggag ccgcacgt                                       28


<210>  22
<211>  34
<212>  DNA
<213>  Artificial Sequence

<220>
<223>  PCR primer

<400>  22
tgaaccggtc tccttctcac gagtccactt gatc                                34


<210>  23
<211>  52
<212>  DNA
<213>  Artificial Sequence

<220>
<223>  PCR primer

<400>  23
ccaaccggtg gatcaggagg tggatcagga ggtatggtga gcaagggcga gg            52


<210>  24
<211>  54
<212>  DNA
<213>  Artificial Sequence

<220>
<223>  PCR primer

<400>  24
ttttcctttt gcggccgctt ttttccttcc ttaggacttg tacagctcgt ccat          54


<210>  25
<211>  28
<212>  DNA
<213>  Artificial Sequence

<220>
<223>  PCR primer

<400>  25
ccaaccggta tcgcatggag ccgcacgt                                          28


<210>  26
<211>  58
<212>  DNA
<213>  Artificial Sequence

<220>
<223>  PCR primer

<400>  26
ttttcctttt gcggccgctt ttttccttcc ttactccttc tcacgagtcc acttgatc        58


<210>  27
<211>  144
<212>  DNA
<213>  Artificial Sequence

<220>
<223>  targeting sequence

<400>  27
ggactcagat ctgccaccat gggctgtggc tgcagctcaa accctgaaga tgactggatg      60

gagaacattg acgtgtgtga gaactgccat tatcccccgc cgaagcttcg aattgatcca     120

ccggatctcg ccaccaccgg tcca                                             144

## Description

The present invention relates to a non-invasive, genetically-encoded fluorescent reporter of Piezo1 activity, applicable to a wide variety of cells and mechanical stimuli.

### Description

Mechanotransduction is a fundamental physiological process that converts environmental stimuli such as compression, tension and shear stress into biochemical signals. This process is critical for almost all mammalian cells. Mechanosensation is essential in a myriad of developmental, physiological and pathophysiological processes including embryogenesis, homeostasis, metastasis and wound healing. How these processes incorporate active feedback via force sensing at the cellular level is an area of active study, and, in recent years, a wide range of tools have been developed to interrogate cell mechanics.

Atomic force microscopy (AFM) and micropipette aspiration have proven to be powerful tools to measure tension in embryos and dissociated cells quantitatively. Other methods, which do not require direct and constant access to the sample, such as droplet-based sensors or optical and magnetic tweezers can modulate probes from a distance and allow precise measurement of molecular to tissue-level forces. Still, these approaches require dissociated tissue and their use is complicated by the requirement of probe injection and the size of the probes, which can damage the tissue.

The necessity to a non-invasive measurement of molecular forces in cells led to the development of genetically-encoded, Forster resonance energy transfer (FRET)-based fluorescent tension sensors capable of measuring mechanical forces across specific cytoskeletal and adhesion proteins such as vinculin, β-spectrin and cadherins. As the specificity and force sensitivity of these probes is defined by the choice of protein and the FRET tension module, their use is restricted to a limited range of biological contexts and force regimes.

Piezo proteins have been identified as ion channels mediating mechanosensory transduction in mammalian cells. They are capable of responding to various external mechanical stimuli. It has been shown that the C-terminus of Piezo1 resides within the cytosol and contains an ion-permeating pore, which has a preference for divalent cations such as calcium (Ca²⁺) over monovalent cations. Upon channel opening, Ca²⁺ concentration near the pore, referred to as Ca²⁺ microdomain, is typically several fold higher than resting levels.

Most vertebrates have two *Piezo* genes - *Piezo1* and *Piezo2.* While Piezo2 function is mainly restricted to the peripheral nervous system, Piezo1 is expressed in a wide range of tissues and has been shown to contribute to mechanotransduction in various organs (Table 1).

**Table 1: A list of biological processes in which Piezo1 is involved.**

| **Biological processes Piezo1 is involved in** |
|---|
| Cardiovascular mechanosensitivity |
| Erythrocyte mechanosensitivity and volume regulation |
| Neural stem cell mechanosensitivity and differentiation |
| Epithelial cell mechanosensitivity |
| Bladder mechanosensitivity |
| Kidney mechanosensitivity |
| Cartilage mechanosensitivity |
| Periodontal cell mechanosensitivity |
| Cell adhesion |
| Cell migration |

Mutations in human Piezo1 gene have been implicated in diseases such as dehydrated hereditary stomacytosis and general lymphatic dysplasia. Global knockout of Piezo1 in mice causes embryonic lethality, highlighting the importance of this channel for development and homeostasis. How cells and tissues integrate Piezo1 activity has been mainly examined by outputs such as cell morphological changes, protein expression, electrophysiological signalling and transcriptional activity in response to mechanical stimuli.

Based on the above-mentioned state of the art, the objective of the present invention is to provide means and methods to a new Piezo1-based fluorescent reporter including genetically-encoded calcium indicator (GECI) with low Ca²⁺ affinity and a wide dynamic range to reliably monitor the dramatic Ca²⁺ changes in the microdomains, while displaying a low response to cytosolic Ca²⁺, which serves as an important secondary messenger in many cellular processes. The inventors evaluated GCaMPs, a class of GECIs, as fluorescent reporters of Piezo1 function. In contrast to FRET-based GECIs, GCaMPs occupy a narrow spectral range that allows a simultaneous imaging of multiple fluorescent markers. Progressive protein engineering efforts have yielded GCaMP variants with a wide dynamic range of responses and with high signal-to-noise ratios (SNR). This objective is attained by the claims of the present specification.

### Terms and definitions

The term *transmembrane pore-forming polypeptide* in the context of the present specification relates to a structural assembly of polypeptide segments that form a pore through a lipid bilayer. In other word, a transmembrane pore is formed, when polypeptide segments cross the lipid bilayer, e.g. the cell membrane and connect the extracellular and intracellular contents. A transmembrane pore-forming polypeptide allows ions of certain size and/or charge to pass through the pore. Some transmembrane pore-forming polypeptides are permeable for more than one type of ion. In many ion-permeable transmembrane pore-forming polypeptides, a passage through the pore is governed by a "gate", which is opened or closed in response to chemical or electrical signals, temperature or mechanical force. A mechanosensitive pore forming-polypeptide responds to a mechanical deformation of the cell membrane or lipid bilayer. Mechanical deformations can include changes in the tension and shear of the cell membrane. A non-limiting example of mechanically sensitive transmembrane pore-forming polypeptide is cation selective Piezo protein family that becomes permeable for calcium ions in response to forces applied to the protein or to the cell membrane.

The term *green fluorescent polypeptide* in the context of the present specification relates to a polypeptide comprising the amino acid sequence of UniProt - P42212. The UniProt number refers to entries in UniProt data base. Green fluorescent polypeptide exhibits bright green fluorescence when exposed to light in the blue to ultraviolet range. The green fluorescent polypeptide has an emission peak of 509 nm and a quantum yield of 0.79.

The term *enhanced green fluorescent polypeptide* in the context of the present specification relates to a derivative of green fluorescent polypeptide with a point mutation of phenylalanine to leucine at position 64 and comprises the amino acid sequence of UniProt - P42212. The enhanced green fluorescent polypeptide has a fluorescence quantum yield of 0.6. The enhanced green fluorescent polypeptide allows a usage of green fluorescent polypeptide in mammalian cells.

Then term *circularly permuted green fluorescent polypeptide* in the context of the present specification relates to a polypeptide derived from the green fluorescent polypeptide that has a reorganized polypeptide chain by circular permutation. A circularly permutated green fluorescent polypeptide is formed by interchanging and reconnecting the amino and carboxyl portions of the green fluorescent polypeptide by a short peptide sequence. The circular permutation of the green fluorescent polypeptide allows the chromophore to be accessible to protons outside of the proteins. The circularly permutated green fluorescent polypeptide is used to transduce the information of an interaction or a mechanical stimulus into a fluorescent signal in an excitation and emission range of 485 - 530nm. A non-limiting example of a circularly permuted green fluorescent polypeptide is comprised within an amino acid sequence encoded by a nucleic acid sequence characterized by SEQ ID 02.

The term *red fluorescent polypeptide* in the context of the present specification relates to a polypeptide comprising the amino acid sequence of UniProt - A0A172WBN6. Red fluorescent protein is optimally excited at the 530nm and detected at 588nm.

The term *enhanced red fluorescent polypeptide* in the context of the present specification relates to a derivative of red fluorescent polypeptide with an advanced red-shifted excitation. The enhanced red fluorescent polypeptide has a reduced autofluorescence, lower light-scattering and lower phototoxicity at longer wavelengths.

The term *blue fluorescent polypeptide* in the context of the present specification relates to a derivative of green fluorescent polypeptide containing the amino acid substitution of tyrosine 66 to histidine comprising the amino acid sequence of UniProt - P80893. The blue fluorescent polypeptide exhibits a broad absorption band in the ultraviolet range close to 380nm and an emission maximum at 448nm.

The term *enhanced blue fluorescent polypeptide* in the context of the present specification relates to a derivative of blue fluorescent polypeptide containing point mutations of phenylalanine 64 to leucine, serine 65 to threonine, tyrosine 66 to histidine and tyrosine 145 to phenylalanine. The enhanced blue fluorescent polypeptide has excitation and emission maxima of 380 and 440 nm, respectively.

The term *yellow fluorescent polypeptide* in the context of the present specification relates to a genetic mutant of green fluorescent polypeptide comprising the amino acid sequence of UniProt - A0A059PIR9. The yellow fluorescent polypeptide has an excitation peak of 514nm and an emission peak of 527nm.

The term *enhanced yellow fluorescent polypeptide* in the context of the present specification relates to a derivative of red fluorescent polypeptide that has a reduced chloride sensitivity, faster maturation, and increased brightness. Non-limiting examples of the enhanced yellow fluorescent polypeptide are Citrine, Venus, and Ypet.

The term *cyan fluorescent polypeptide* in the context of the present specification relates to a derivative of green fluorescent polypeptide comprising the amino acid sequence of UniProt - A0A059PIU2. Cyan fluorescent polypeptide contains an amino acid substitution of tyrosine 66 to tryptophan. Cyan fluorescent polypeptide can be excited at the 405 nm and is optimally detected at 485 nm.

The term *enhanced cyan fluorescent polypeptide* in the context of the present specification relates to a derivative of cyan fluorescent polypeptide possessing a quantum yield of 0.36. Non-limiting examples of the enhanced cyan fluorescent polypeptide are Cerulean, CyPet and mTurquoise2.

The term *shear force* in the context of the present specification relates to the influence of flow and the associated shear stress on a cell. The molecular basis of a shear force involves receptor/ligand interactions and/or ion transport though the cell membrane. Shear force includes processes that are responsible for sensing fluid flow and mechanical force. On a single cell scale a useful unit is the pNewton, and a single motor protein produces a maximal force of few pNewtons.

The term *fluid shear force* in the context of the present specification relates to a kind of shear force that is mediated by fluid flow. A lipid bilayer or a cell membrane sensitive for fluid shear stress harbours shear-sensing mechanisms. A non-limiting example of fluid shear stress and its sensing mechanism is the application of a pump system (ibidi) to the cells expressing Piezo1 protein. A non-limiting example of the applied fluid shear forces are in the range of 1-5 dyne/cm² and 5-30 dyne/cm².

The term *pressure force* in the context of the present specification relates to an external mechanical force that is applied to a cell. A pressure force can affect cell growth, signal transduction, gene expression and ion transport. A non-limiting example of a pressure force is the hydrostatic pressure.

The term *cyclic pressure* in the context of the present specification relates to a kind of pressure stress that is applied to a cell. A non-limiting example of a cyclic pressure is the cyclic hydrostatic pressure on human bone marrow-derived mesenchymal progenitor cells that exhibit an increase in F-actin stress fibre formation in response to cyclic pressure.

The term *stretch force* in the context of the present specification relates to an external force that is applied to a cell. The range of stretch force applied to a cell varies on the basis of the cell type and growing conditions. The stretch force can lead to cytoskeleton reorganization and strengthening of the focal adhesions due to the cellular tendency to resist the deformation caused by force and to maintain optimum mechanical conditions. A non-limiting example of stretch force is the static stretching of vascular smooth muscle cells cultured on a soft substrate (∼5 kPa) that induces changes in cytoskeleton contractility.

The term *cyclic stretch* in the context of the present specification relates to a kind of stretch force. The cell responses to cyclic stretch conditions depend on cell type and stretch regime. A non-limiting example of cyclic stretch applied to a cell is at frequencies ranging from 0.01 to 10 Hz that can cause a significant increase in spreading and stress fibre formation, with the optimum at 0.1 Hz.

Amino acid sequences are given from amino to carboxyl terminus. Capital letters for sequence positions refer to L-amino acids in the one-letter code (Stryer, Biochemistry, 3rd ed. p. 21). Lower case letters for amino acid sequence positions refer to the corresponding D- or (2R)-amino acids.

The term "nucleotides" in the context of the present invention are nucleic acid or nucleic acid analogue building blocks, oligomers of which are capable of forming selective hybrids with RNA or DNA oligomers on the basis of base pairing. The term nucleotides in this context includes the classic ribonucleotide building blocks adenosine, guanosine, uridine (and ribosylthymine), cytidine, the classic deoxyribonucleotides deoxyadenosine, deoxyguanosine, thymidine, deoxyuridine and deoxycytidine. It further includes analogues of nucleic acids such as phosphotioates, 2'O-methylphosphothioates, peptide nucleic acids (PNA; N-(2-aminoethyl)-glycine units linked by peptide linkage, with the nucleobase attached to the alpha-carbon of the glycine) or *locked nucleic acids* (LNA; 2'O, 4'C methylene bridged RNA building blocks). The hybridizing sequence may be composed of any of the above nucleotides, or mixtures thereof.

In certain embodiments, the hybridizing sequence is at least 80% identical, more preferred 85%, 90%, 92%, 94%, 95%, 96%, 97%, 98% or 99% identical to the reverse complimentary sequence of SEQ ID 1 or SEQ ID 2. In certain embodiments, the hybridizing sequence comprises deoxynucleotides, phosphothioate deoxynucleotides, LNA and/or PNA nucleotides or mixtures thereof.

In the context of the present specifications the terms *sequence identity* and *percentage of sequence identity* refer to the values determined by comparing two aligned sequences. Methods for alignment of sequences for comparison are well-known in the art. Alignment of sequences for comparison may be conducted by the local homology algorithm of Smith and Waterman, Adv. Appl. Math. 2:482 (1981), by the global alignment algorithm of Needleman and Wunsch, J. Mol. Biol. 48:443 (1970), by the search for similarity method of Pearson and Lipman, Proc. Nat. Acad. Sci. 85:2444 (1988) or by computerized implementations of these algorithms, including, but not limited to: CLUSTAL, GAP, BESTFIT, BLAST, FASTA and TFASTA. Software for performing BLAST analyses is publicly available, e.g., through the National Center for Biotechnology-Information (http://blast.ncbi.nlm.nih.gov/).

One example for comparison of amino acid sequences is the BLASTP algorithm that uses the default settings: Expect threshold: 10; Word size: 3; Max matches in a query range: 0; Matrix: BLOSUM62; Gap Costs: Existence 11, Extension 1; Compositional adjustments: Conditional compositional score matrix adjustment. One such example for comparison of nucleic acid sequences is the BLASTN algorithm that uses the default settings: Expect threshold: 10; Word size: 28; Max matches in a query range: 0; Match/Mismatch Scores: 1.-2; Gap costs: Linear. Unless otherwise stated, sequence identity values provided herein refer to the value obtained using the BLAST suite of programs (Altschul et al., J. Mol. Biol. 215:403-410 (1990)) using the above identified default parameters for protein and nucleic acid comparison, respectively.

A first aspect of the invention relates to a polypeptide that can comprise a first polypeptide module. The first polypeptide module can contain a transmembrane pore-forming polypeptide, which is permeable for calcium ions.

The permeability of the pore-forming polypeptide can be modulated by a mechanical stimulus. Non-limiting examples of the mechanical stimuli are shear force, fluid shear force, pressure force, cyclic pressure, stretch force and cyclic stretch force. In addition to the first polypeptide module, the polypeptide can contain a second polypeptide module.

The permeability of the pore-forming polypeptide may alternatively be modulated by voltage, concentration of H+ ions (pH), or a particular ligand. The general functionality of Ca permeability of any calcium permeable channel can be employed in the polypeptide invention.

The second polypeptide module comprises a luminescent polypeptide, which can be a fluorescent polypeptide. The second polypeptide segment can also contain a calcium binding polypeptide. A non-limiting example of a calcium ion polypeptide is characterized by dissociation constant K_{D} in the ranges of 100 nmol/L to 10 µmol/L and/or 500 nmol/L to 2 µmol/L. Additionally, the second polypeptide segment can comprise a modulator peptide sequence. The modulator peptide sequence is capable to bind to the calcium binding polypeptide with a higher affinity in the calcium-bound state than in the absence of calcium ions. In certain embodiments, the second polypeptide module is one continuous polypeptide chain, wherein and the elements exhibit covalent bonding.

In certain embodiments, the luminescence of the luminescent polypeptide can alter upon binding of the modulator peptide sequence to the calcium binding polypeptide, when the calcium binding polypeptide is in a calcium-bound state.

In certain embodiments, a peptide linker sequence can covalently connect the first polypeptide module and the second polypeptide module.

In certain embodiments, the transmembrane pore-forming polypeptide is permeable for calcium ions. A non-limiting example of transmembrane pore-forming polypeptide that is permeable for calcium ions can comprise the amino acid sequence of SEQ ID 01 or a functional equivalent thereof being ≥85%, 90%, 92%, 94%, 96%, particularly 98% or 99%, identical to SEQ ID 01.

In certain embodiments, the luminescent polypeptide is a fluorescent polypeptide. Non-limiting examples of a fluorescent polypeptide are green fluorescent polypeptide, enhanced green fluorescent polypeptide, circularly permuted green fluorescent polypeptide, red fluorescent polypeptide, enhanced red fluorescent polypeptide, blue fluorescent polypeptide, enhanced blue fluorescent polypeptide, yellow fluorescent polypeptide, enhanced yellow fluorescent polypeptide, cyan fluorescent polypeptide and enhanced cyan fluorescent polypeptide. Fluorescent proteins are disclosed inter alia in US6172188, US2009117650, US2010167394, US2010184116, US2010167394, US2009017535, US2003175859, all of which are incorporated herein by reference.

In certain embodiments, the luminescent polypeptide can comprise the amino acid sequence of SEQ ID 02 or a functional equivalent thereof being ≥85%, 90%, 92%, 94%, 96%, 98% or 99%, identical to SEQ ID 02 (Nagai et al., Proc Nat Acad Sci USA 2001 98(6), 3197202).

In particular embodiments, the luminescent polypeptide moiety is located adjacent (more particularly C-terminally / 3') to the modulator peptide, as exemplarily shown in SEQ ID No 15.

In certain embodiments, the calcium binding polypeptide can comprise the amino acid sequence of SEQ ID 03 or a functional equivalent thereof being ≥85%, 90%, 92%, 94%, 96%, 98% or 99%, identical to SEQ ID 03. This sequence is a mutated calmodulin having an affinity to Ca that moderated by the modulator peptide.

The skilled person is aware that there are other calcium indicators to choose from in practicing the present invention. As one non-limiting example, such calcium indicator may be based on another calcium sensitive protein such as troponin C, and variations thereof (see Mank et al., Biophys. J. 90(5) 1790-1796 (2006)).

In certain embodiments, the modulator peptide sequence can have an amino acid length of 5 to 30 amino acids and/or 10 to 25 amino acids. Furthermore, the modulator peptide sequence is capable of forming a hydrophobic interaction with the calcium binding polypeptide.

In certain embodiments, the modulator peptide sequence can comprise the amino acid sequence selected from SEQ ID 04, SEQ ID 05, SEQ ID 06, SEQ ID 07, SEQ ID 08, SEQ ID 09, SEQ ID 10, SEQ ID 11, SEQ ID 12, or a functional equivalent thereof being ≥85%, 90%, 92%, 94%, 96%, 98% or 99%, identical to SEQ ID 04.

In certain embodiments, the second polypeptide module comprises or consists of the amino acid sequence of SEQ ID 13 or a functional equivalent thereof being ≥85%, 90%, 92%, 94%, 96%, 98% or 99%, identical to SEQ ID 13.

In certain embodiments, the polypeptide comprises or consists of the amino acid sequence of SEQ ID 14 or a functional equivalent being ≥85%, 90%, 92%, 94%, 96%, 98% or 99%, identical to SEQ ID 14.

In certain embodiments, the peptide linker sequence can be characterized by its length. Non-limiting examples of a peptide linker can have a size range of 1 to 10 amino acids residues, 2 to 8 amino acids residues or can contain 4 amino acids residues.

In certain embodiments, the peptide linker sequence can be characterized by the amino acid sequence. A non-limiting example of the peptide linker sequence can comprise glycine and serine residues or can have an amino acid sequence of Gly-Ser-Gly-Gly.

Another aspect of the invention relates to a polynucleotide sequence that encodes the above-mentioned polypeptide sequence.

Another aspect of the invention relates to a recombinant expression vector that can comprise the above-mentioned polynucleotide sequence. The polynucleotide sequence can be under control of a promotor sequence operable in a host cell that can be a mammalian host cell.

Another aspect of the invention relates to a host cell that can comprise the above-mentioned polynucleotide sequence or the above-mentioned recombinant expression vector.

In certain embodiments the host cell can be a eukaryotic cell or a mammalian cell.

Another aspect of the invention relates to a method for detection of a stimulus applied to a cell. The method can comprise the following steps:
(i) providing a host cell that can comprise the above-mentioned polynucleotide sequence or the above-mentioned recombinant expression vector,
(ii) determining a fluorescence of the host cell.

In certain embodiments, the fluorescence can change in dependence of a stimulus applied to a host cell.

In certain embodiments, the stimulus applied to a cell can be a mechanical stimulus.

In certain embodiments, the method for detection of a stimulus applied to a cell further comprises the step of applying a mechanical stimulus to the host cell.

In certain embodiments, the mechanical stimulus can comprise a mechanical force stimulus. Non-limiting examples of a mechanical stimulus are shear force, fluid shear force, pressure force, cyclic pressure, stretch force and cyclic stretch.

In certain embodiments, the method for detection of a stimulus applied to a cell is detectable by a fluorescence that can be measured by an excitation at 488nm. A change of measured fluorescence is detected after applying a mechanical stimulus in a range of 0 to 400nN or in a range of 0-20 dyne/cm² over a time period of 100 to 200 msec.

Another aspect of the invention relates to a method to assess the ability of a test compound to modulate the permeability of transmembrane pore-forming polypeptide permeable for a calcium ion. Therein, the transmembrane pore-forming polypeptide permeable for a calcium ion is comprised in a polypeptide according to any of the embodiments of the first aspect of the invention described herein. The method according to the invention is performed on a cell comprising the polypeptide of the invention in presence and absence of the test compound, and modulation of said transmembrane pore-forming polypeptide is assessed by comparison of results. If the calcium channel response is significantly changed in presence of the test compound, it can be categorized as a possible drug candidate for modulating the physiological response associated with the channel.

The invention further relates to the following items, without being limited to them:
Item 1: A polypeptide comprising:
   a. a first polypeptide module comprising a transmembrane pore-forming polypeptide permeable for a calcium ion, wherein the permeability of said pore-forming polypeptide is modulated by a stimulus, particularly a mechanical stimulus;
   b. a second polypeptide module comprising
      - a luminescent polypeptide, particularly a fluorescent polypeptide,
      - a calcium binding polypeptide capable of selectively binding a calcium ion, particularly wherein the binding of said polypeptide to said calcium ion is characterized by a dissociation constant K_{D} in the range of 100 nmol/L to 10 µmol/L, more particular said K_{D} is in the range 500 nmol/L to 2 µmol/L, and
      - a modulator peptide sequence capable of binding to said calcium binding polypeptide, wherein
         - said modulator peptide sequence has a higher affinity to said calcium binding polypeptide when said calcium binding sequence is bound to said calcium ion than when said calcium binding sequence is not bound to said calcium ion,
         - and wherein luminescence of said luminescent polypeptide alters upon binding of said modulator peptide sequence to said calcium binding polypeptide
   c. a peptide linker sequence covalently connecting said first polypeptide module and said second polypeptide module.
Item 2: The polypeptide according to item 1, wherein said transmembrane pore-forming polypeptide permeable for a calcium ion comprises or consists of the amino acid sequence of SEQ ID 01 or a functional equivalent thereof being ≥85%, 90%, 92%, 94%, 96%, particularly 98% or 99%, identical to SEQ ID 01.
Item 3: The polypeptide according to any one of preceding items, wherein said luminescent polypeptide is a fluorescent polypeptide selected from
   (i) a green fluorescent polypeptide, particularly an enhanced green fluorescent polypeptide, more particularly a circularly permuted green fluorescent polypeptide;
   (ii) a red fluorescent polypeptide, particularly an enhanced red fluorescent polypeptide;
   (iii) a blue fluorescent polypeptide, particularly an enhanced blue fluorescent polypeptide;
   (iv) a yellow fluorescent polypeptide, particularly an enhanced yellow fluorescent polypeptide;
   (v) a cyan fluorescent polypeptide, particularly an enhanced cyan fluorescent polypeptide.
Item 4: The polypeptide according to any one of preceding items, wherein said luminescent polypeptide is comprised within an amino acid sequence encoded by a nucleic acid sequence characterized by SEQ ID 02 or a functional equivalent thereof being ≥85%, 90%, 92%, 94%, 96%, particularly 98% or 99%, identical to said amino acid sequence encoded by a nucleic acid sequence characterized by SEQ ID 02;
   particularly wherein said luminescent polypeptide is comprised within an amino acid sequence encoded by a nucleic acid sequence characterized by SEQ ID 15 or a functional equivalent thereof being ≥85%, 90%, 92%, 94%, 96%, particularly 98% or 99%, identical to said amino acid sequence encoded by a nucleic acid sequence characterized by SEQ ID 15, wherein said amino acid sequence further comprises said modulator peptide sequence.
Item 5: The polypeptide according to any one of preceding items, wherein said calcium binding polypeptide comprises or consists of an amino acid sequence encoded by a nucleic acid sequence characterized by SEQ ID 03 or a functional equivalent thereof being ≥85%, 90%, 92%, 94%, 96%, particularly 98% or 99%, identical to said amino acid sequence encoded by a nucleic acid sequence characterized by SEQ ID 03.
Item 6: The polypeptide according to any one of preceding items, wherein said modulator peptide sequence has an amino acid length of 5 to 30 amino acids, particularly a length of 10 to 25 amino acids and wherein said modulator peptide sequence is capable of forming a hydrophobic interaction with said calcium binding polypeptide.
Item 7: The polypeptide according to any one of preceding items, wherein said modulator peptide sequence comprises or consists of the amino acid sequence selected from SEQ ID 04, SEQ ID 05, SEQ ID 06, SEQ ID 07, SEQ ID 08, SEQ ID 09, SEQ ID 10, SEQ ID 11, SEQ ID 12, or a functional equivalent thereof being ≥85%, 90%, 92%, 94%, 96%, particularly 98% or 99%, identical to SEQ ID 04.
Item 8: The polypeptide according to any one of preceding items, wherein said second polypeptide module comprises or consists of an amino acid sequence encoded by a nucleic acid sequence characterized by SEQ ID 13 or a functional equivalent thereof being ≥85%, 90%, 92%, 94%, 96%, particularly 98% or 99%, identical to said amino acid sequence encoded by a nucleic acid sequence characterized by SEQ ID 13.
Item 9: The polypeptide according to any one of preceding items, wherein said polypeptide comprises or consists of an amino acid sequence encoded by a nucleic acid sequence characterized by of SEQ ID 14 or a functional equivalent being ≥85%, 90%, 92%, 94%, 96%, particularly 98% or 99%, identical to said amino acid sequence encoded by a nucleic acid sequence characterized by SEQ ID 14.
Item 10: The polypeptide according to any one of preceding items, wherein said peptide linker sequence is characterized by
   (i) a length in the range of 1 to 10 amino acids residues, particularly 2 to 8 amino acids residues, more particular 4 amino acids residues, and/or
   (ii) an amino acid sequence comprising glycine and serine residues, particularly an amino acid sequence of Gly-Ser-Gly-Gly.
Item 11: A polynucleotide sequence encoding the polypeptide according to any one of preceding items.
Item 12: A recombinant expression vector comprising a polynucleotide sequence according to item 10, wherein said polynucleotide sequence is under control of a promotor sequence operable in a host cell, particularly a mammalian host cell.
Item 13: A host cell comprising a polynucleotide sequence according to item 10 or a recombinant expression vector according to item **Fehler! Verweisquelle konnte nicht gefunden werden..**
Item 14: The host cell according to item 13, wherein said host cell is a eukaryotic cell, particularly a mammalian cell.
Item 15: A method for detection of a stimulus applied to a cell, comprising the steps of:
   (i) providing a host cell according to items 13 and/or **Fehler! Verweisquelle konnte nicht gefunden werden.,**
   (ii) determining a fluorescence of said host cell,
   wherein said fluorescence changes in dependence of said stimulus applied to said host cell.
Item 16: The method according to item 11, wherein said stimulus is a mechanical stimulus.
Item 17: The method according to items 15 and 12, further comprising the step applying a mechanical stimulus to said host cell.
Item 18: The method according to items 15 to 13, wherein said mechanical stimulus comprises a mechanical force stimulus selected from:
   (i) shear force, particularly fluid shear force,
   (ii) pressure force, particularly cyclic pressure,
   (iii) stretch force, particularly cyclic stretch.
Item 19: The method according to items 11 to 13, wherein said fluorescence is measured by an excitation at 488nm and a change of measured fluorescence is detected after applying said mechanical stimulus in a range of 0 to 400nN or in a range of 0-20 dyne/cm² over a time period of 100 to 200 msec.
Item 20: A method to assess the ability of a test compound to modulate the permeability of transmembrane pore-forming polypeptide permeable for a calcium ion, wherein said transmembrane pore-forming polypeptide permeable for a calcium ion is comprised in a polypeptide according to any one of items 1 to 10, and a method according to any one of items 15 to 19 is performed in presence and absence of said test compound, and modulation of said transmembrane pore-forming polypeptide is assessed by comparison of results obtained by said method in presence and absence of said test compound.

The invention is further illustrated by the following examples and figures, from which further embodiments and advantages can be drawn. These examples are meant to illustrate the invention but not to limit its scope.

### Brief description of the figures

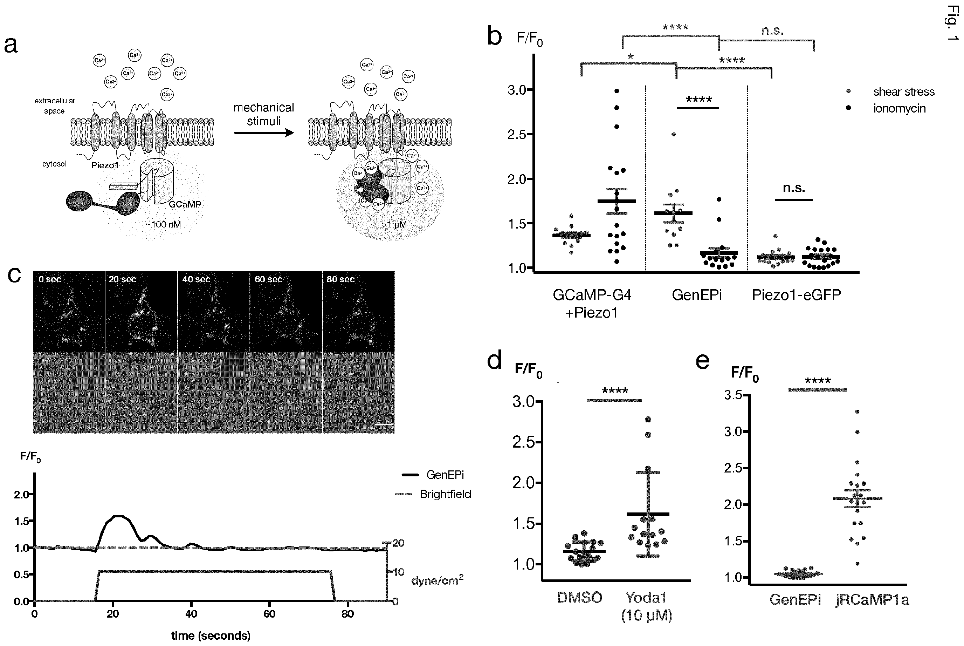

- Fig. 1: shows the functional specificity of the reporter by *in vitro* characterization of GenEPi. (a) GenEPi sensing mechanism. GCaMP is targeted near the C-terminal Piezo1 pore. When mechanical stimuli induce channel opening, incoming Ca2+ (in yellow) binds to GCaMP, causing an increase in green fluorescence. (b) Response of co-transfected Piezo1 and GCaMP-G4, GenEPi, and Piezo1-eGFP to shear stress and ionomycin. GenEPi (n=12, shear stress; n=15, ionomycin) has functional specificity, determined by increased baseline-normalized fluorescence signal (F/F0) to shear stress, compared to cytosolic GCaMP-G4 (n=13, shear stress; n=18, ionomycin) and low F/F0 to ionomycin, similar to levels measured with Piezo1-eGFP (n=16, shear stress, n=19, ionomycin). Error bar=SEM; data from three independent experiments. Two tailed Mann-Whitney test, ****= p<0.0001; *=p<0.05; n.s.=p>0.05. (c) Representative example of GenEPi activation and F/F0 signal intensity profile (black) in response to 10 dyne/cm2 shear stress (blue). (d) F/F0 signal response of GenEPi to 10 µM Yoda1 (n=8) or DMSO (n=11). Mann-Whitney test, *=p<0.05, error bar=SEM, data from three independent experiments. (e) F/F0 signal response of GenEPi and jRCaMP1a (n=19) to 30 µM ATP. Welch's t-test, ****= p<0.0001, error bar=SEM, data from six independent experiments. Scale bar, 10 µm (c).

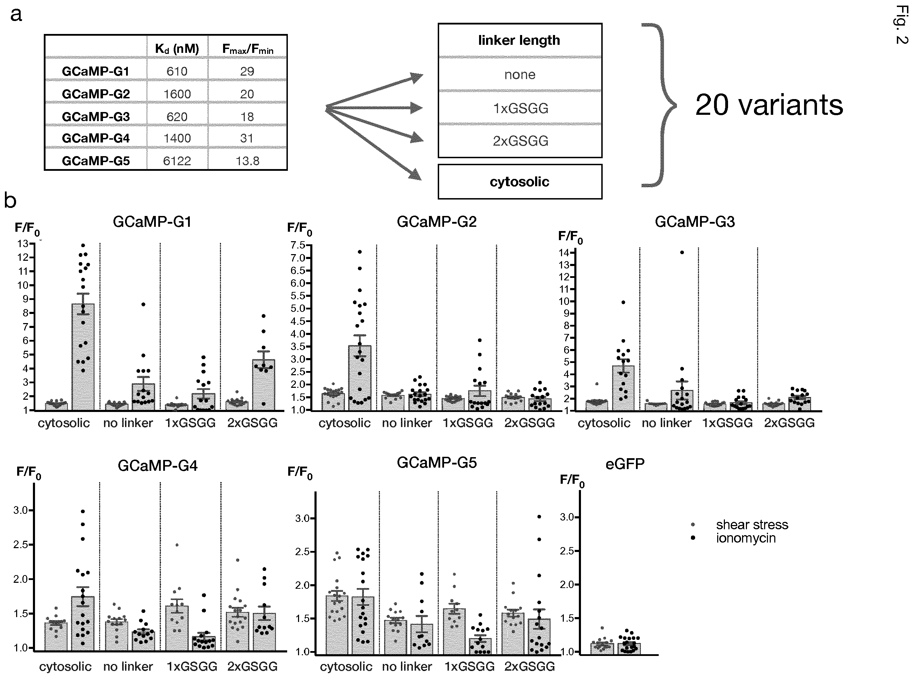

- Fig. 2: shows the design and results of the screen to identify a reporter for mechanical stimuli. (a) Design of the screen. Five GCaMPs that possess a low Ca2+ affinity with various dynamic ranges (Fₘₐₓ/Fₘᵢₙ) were attached to the C-terminus of human Piezo1 without any linker, with the addition of a short linker (i.e. 1xGly-Ser-Gly-Gly (GSGG) linker sequence) and long linker (i.e. 2xGSGG linker sequence), respectively. The response of the resulting 20 variants (including the co-transfected cytosolic GCaMPs and human Piezo1) to ionomycin (1 µM) and shear stress (10 dyne/cm2) was tested. (b) Results of the screen. The shear stress (blue) and ionomycin (black) response are shown for all tested variants. eGFP fused to human Piezo1 serves as a control for the noise acquired during imaging under either stimulus. Error bar=s.e.m., n=3 independent experiments, please see Supplementary table 3 for detailed statistical information.

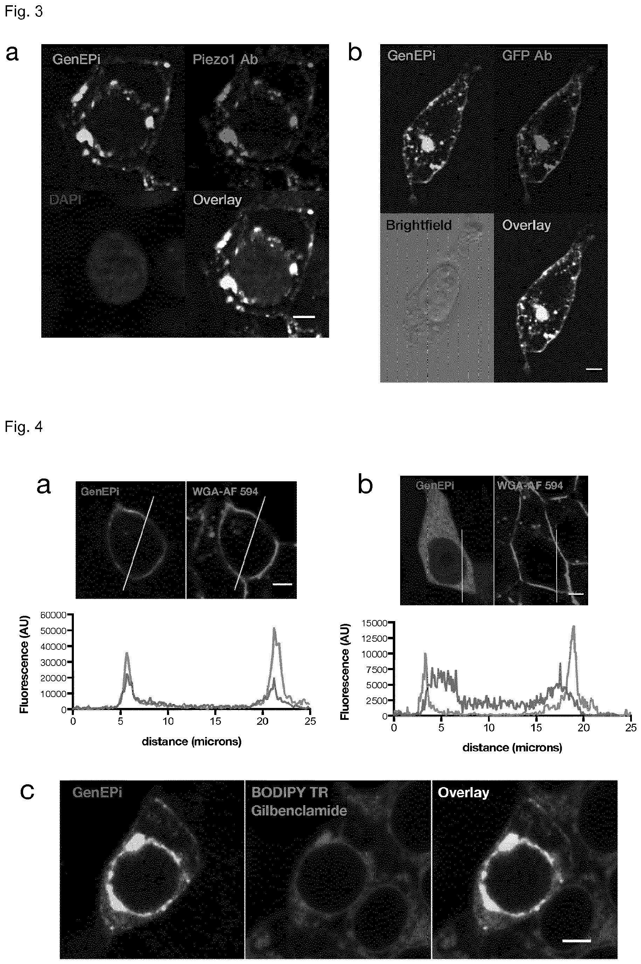

- Fig. 3: shows protein integrity of GenEPi. (a) GenEPi (green) expressing HEK 293T cell stained with antibody against Piezo1 (magenta) and DAPI (blue). (b) GenEPi (green) expressing HEK 293T cell in brightfield image, stained with antibody against GFP (magenta) which binds to GCaMP. Scale bar, 5 µm.

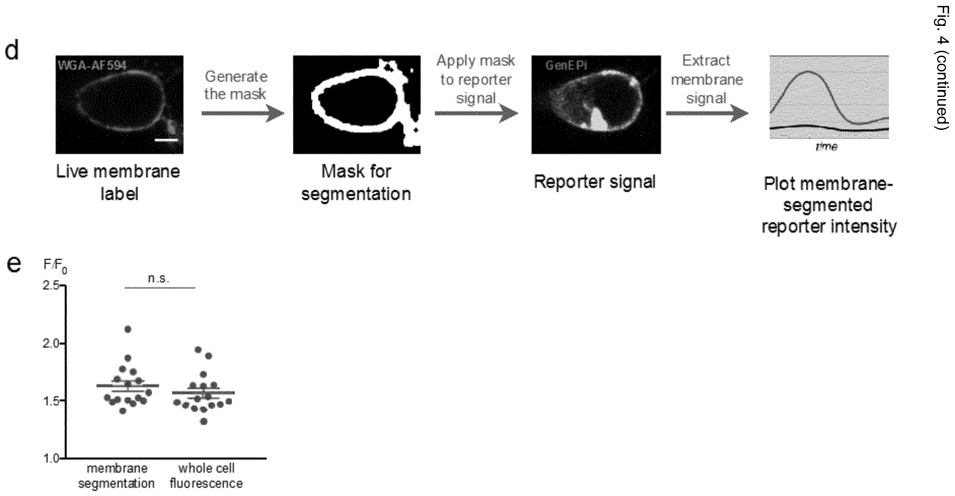

- Fig. 4: shows the intracellular localization of GenEPi and validation of whole cell fluorescence measurement. (a) Fluorescence signal intensity profiles of a cross-section through a representative cell expressing GenEPi (green) stained with Wheat Germ Agglutinin conjugated Alexa Fluor 594 (magenta). (b) Process as in a, for HEK293T cells expressing GCaMP-G4. (c) GenEPi expressing (green) HEK 293T cell stained with BODIPY TR Gilbenclamide (magenta), a live stain for ER. (d) Segmentation procedure to obtain plasma membrane masks: live plasma membrane stain (magenta) is used as a mask to extract membrane signals from GenEPi expressing (green) cell. Obtained signals are then plotted over time. (e) Comparison of segmented membrane signals to whole cell signals. n=16 cells, Mann-Whitney test, error bar= s.e.m., n.s=p>0.05. Scale bar, 5 µm (a-d).

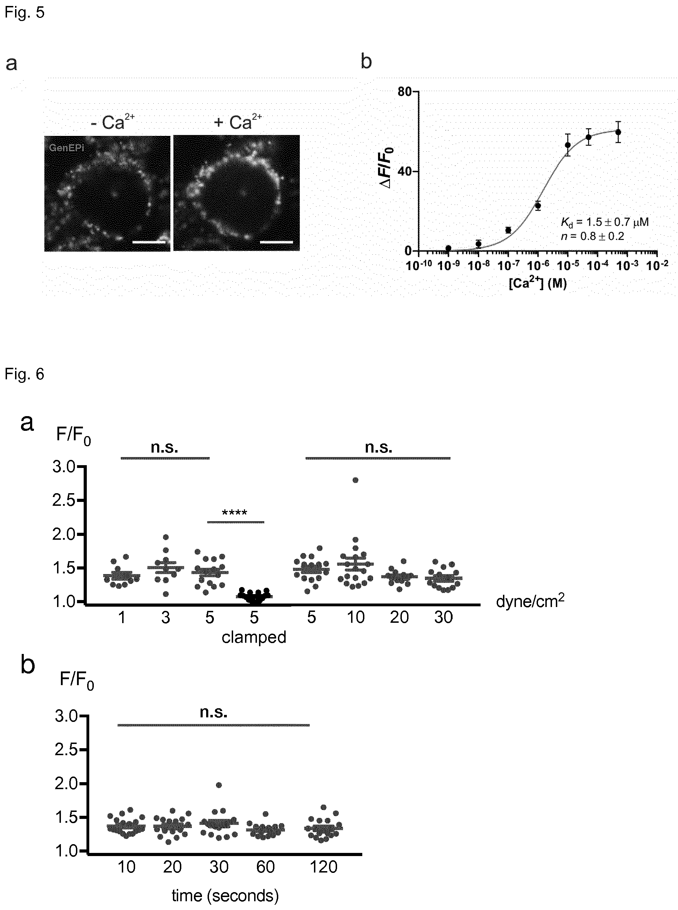

- Fig. 5: shows the *in situ* Ca²⁺ titration of GenEPi in HeLa cells. (a) Representative confocal images of HeLa cells expressing GenEPi showing plasma membrane localization and fluorescence increase in the presence of 100 µM Ca²⁺. (b) In situ Ca²⁺ titration of GenEPi. Ca²⁺-dependent changes in fluorescence intensities were fitted to the Hill equation to estimate K_{d} (dissociation constant) and n (Hill coefficient). Fluorescence dynamic range (Fₘₐₓ-F₀)/F₀ or ΔF/F₀ was expressed as mean ± s.e.m. (12 to 42 cells from at least three independent experiments). Scale bar, 10 µm (a).
- Fig. 6: shows that the level or duration of applied fluid shear stress does not affect the response amplitude. (a) F/F₀ response of GenEPi transfected HEK293T cells exposed for 60 seconds to different levels of shear stress, ranging from 1-30 dyne/cm², along with a control at 5 dyne/cm² with preventing liquid flow by clamping the tubing. For 1 dyne/cm² (n=10), 3 dyne/cm² (n=10), and 5 dyne/cm² (n=15), One-way ANOVA test was used. For 5 dyne/cm² clamped vs. free flow, Welch's t-test. For 5 dyne/cm² (n=15), 10 dyne/cm² (n=18), 20 dyne/cm² (n=13), and 30 dyne/cm² (n=14), Kruskal-Wallis test, was used. ****=p<0.0001, n.s=p>0.05. Error bar=SEM, data from three independent experiments. (b) F/F₀ response of GenEPi transfected HEK293T cells exposed to 10 dyne/cm² shear stress for 10 seconds (n=19), 20 seconds (n=16), 30 seconds (n=17), 60 seconds (n=15), and 120 seconds (n=18). Kruskal-Wallis test, n.s=p>0.05. Error bar=SEM, data from four independent experiments.

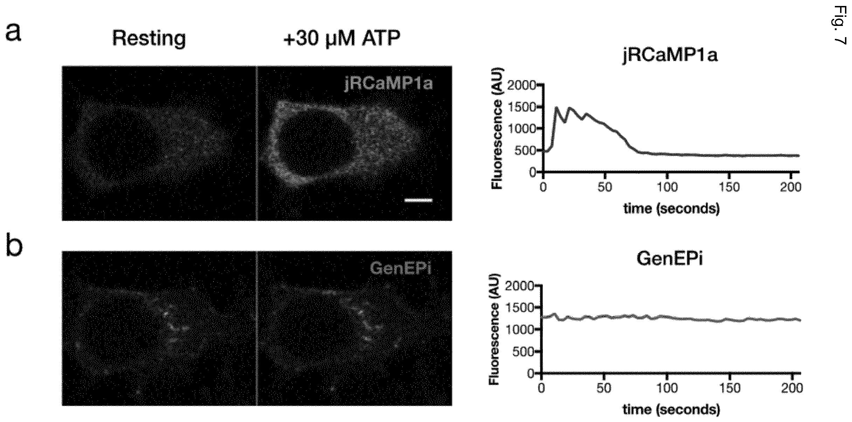

- Fig. 7: shows the response of GenEPi and jRCaMP1a to 30 µM ATP. (a) Representative image of jRCaMP1a (magenta) expressing HEK293T cell in the absence or presence of 30 µM ATP, along with a fluorescence intensity plotted over time. (b) Representative image of the same cell, showing the signal of GenEPi (green) in response to 30 µM ATP, along with the fluorescence intensity plotted over time. Scale bar, 5 µm.

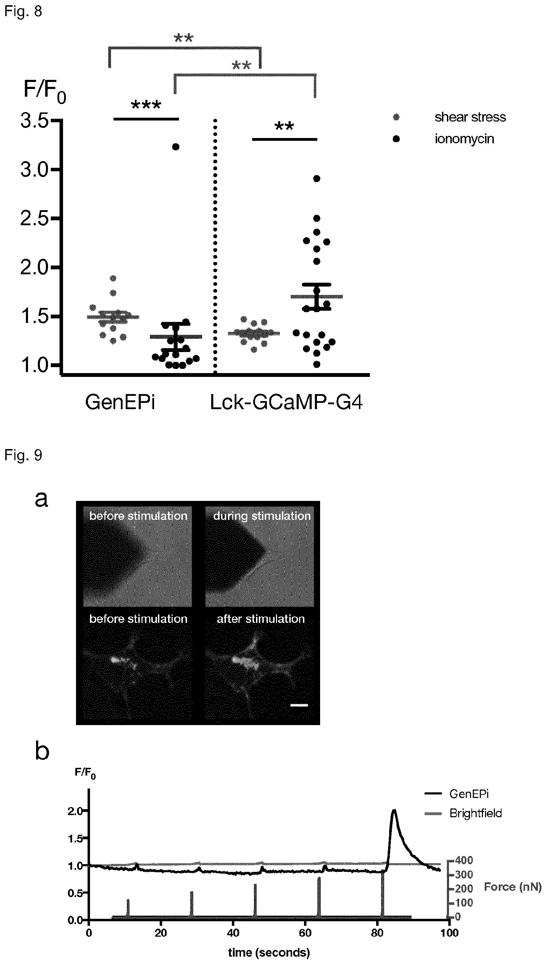

- Fig. 8: shows that the plasma membrane localization alone does not confer functional specificity. Responses of GenEPi and Lck-GCaMP-G4 to 10 dyne/cm² fluid shear stress (n=13 cells) and 1 µM ionomycin (n=16-20 cells). GenEPi shear vs. GenEPi ionomycin and GenEPi vs Lck-GCaMP-G4 ionomycin response, Mann-Whitney test, **=p<0.01, ***=p<0.001. Lck-G4 shear vs. GenEPi shear response and Lck-G4 shear vs. Lck-G4 ionomycin Welch's t-test, **=p<0.01. Error bar=SEM, data from 4 independent experiments.

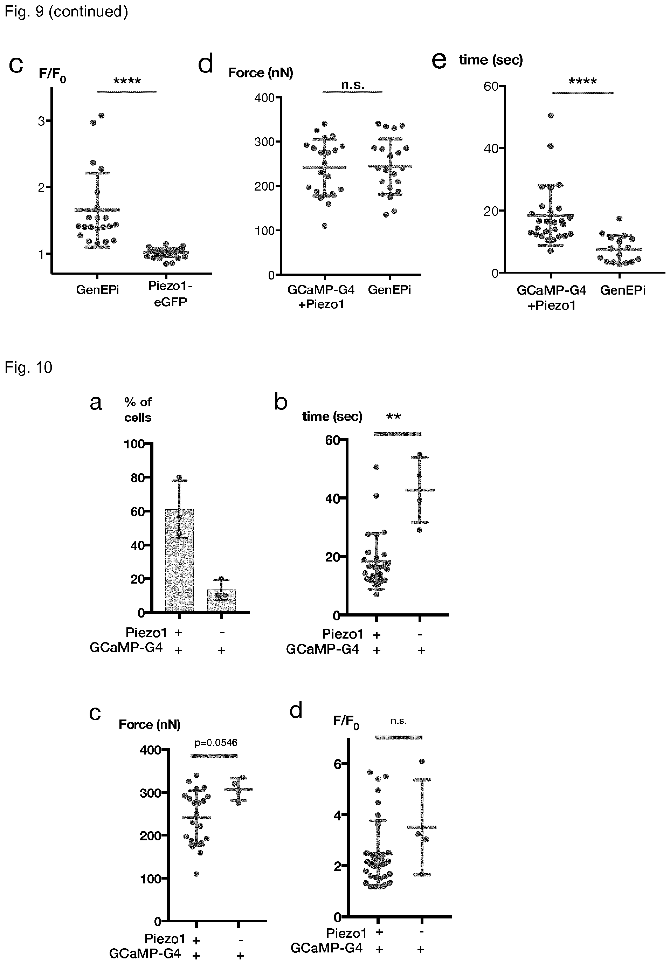

- Fig. 9: shows force sensitivity and increased temporal resolution of GenEPi in response to compressive forces. (a) Representative images of AFM cantilever stimulation of GenEPi expressing HEK293T cells. Top row: bright-field image of cantilever position before and during stimulation; bottom row: fluorescent image of the stimulated cell before and after stimulation. (b) The mechanical stimulation procedure, of compressive forces ranging from 129-370 nN (purple) along with the bright field (grey) and fluorescent (black) traces from the cell depicted in a. (c) Amplitude of optical calcium responses from GenEPi (n=21), and Piezo-eGFP (n=45). Mann-Whitney test,****= p<0.0001. (d) Threshold force levels for human Piezo1 co-transfected with cytosolic GCaMP-G4 (n=21), n.s.=p>0.05, Unpaired t-test (e) Durations of optical calcium responses from cells co-transfected with cytosolic GCaMP-G4 and human Piezo1 (n=27) and GenEPi (n=16). Mann-Whitney test, ****=p<0.0001. Error bar=SEM, data from three independent experiments. Scale bar, 10 µm.
- Fig. 10: shows that the response of HEK 293T cells to compressive forces is dependent on Piezo1 over expression. (a) Percentage of cells transfected with cytosolic GCaMP-G4 in the absence or presence of human Piezo1 over-expression that responded to mechanical stimulation using AFM in each experiment, n=3 experiments. (b) Durations of optical calcium responses or cells transfected with GCaMP-G4 alone (n=4) or co-transfected with human Piezo1 (n=27), Mann-Whitney test, **=p<0.01. (c) Threshold force levels for cells transfected with GCaMP-G4 alone (n=4) or co-transfected with human Piezo1 (n=21), Unpaired t-test, p=0.0546. (d) Amplitudes of optical calcium responses or cells transfected with GCaMP-G4 alone (n=4) or co-transfected with human Piezo1 (n=34), Mann-Whitney test, n.s.=p>0.05. Error bar=SEM, data from n=3 experiments.

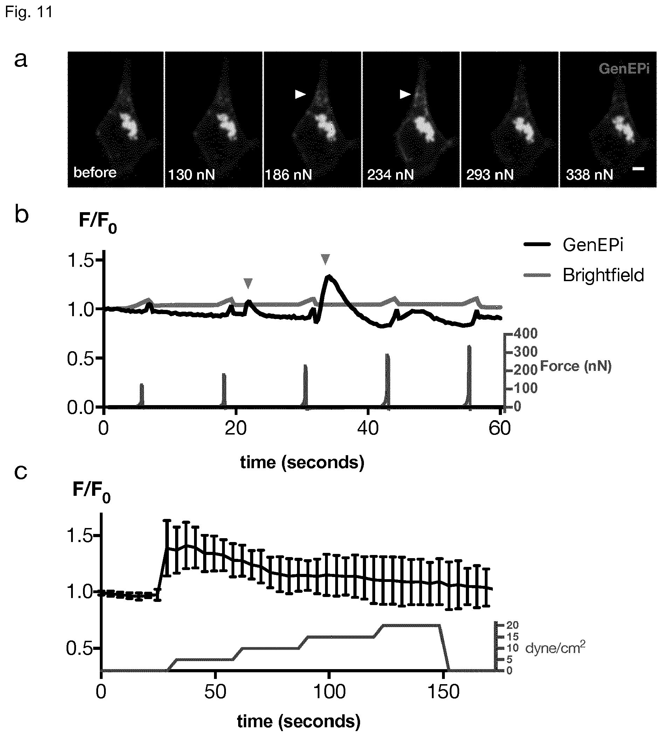

- Fig. 11: shows that the response to repeated stimuli depends on the type of stimulus. (a) Representative image of a graded GenEPi activation and (b) F/F0 signal intensity profile in response to increasing level of mechanical stimulation using AFM. The response of the cell (in black) for each stimulation step (purple) is shown in a. The white arrow heads in a correspond to the peaks pointed with the red arrow heads in b. (c) F/F0 signal intensity profile of GenEPi in response to increasing level (5-10-15-20 dyne/cm²) of fluid shear stress, n=11, error bar=s.e.m., data from three independent experiments. Scale bar, 5µm (a).

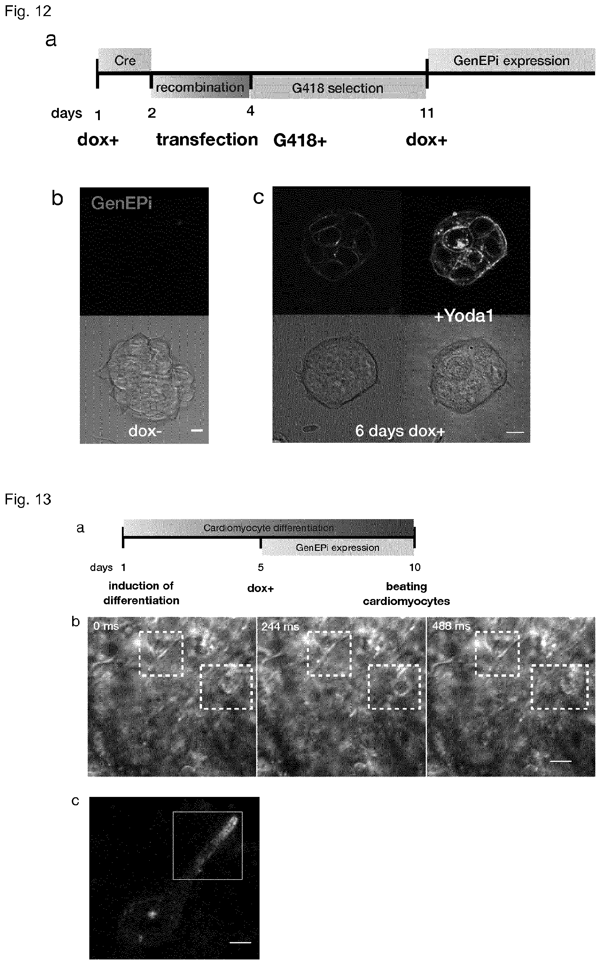

- Fig. 12: shows the generation of doxycycline-inducible GenEPi mESC line and its response to Yoda1. (a) HPRT locus inducible cassette exchange strategy involves the induction of Cre by doxycycline (dox) in transiently transfected cells allowing recombination. Positive clones are selected using G418. Doxycycline induces transgene expression of surviving. (b) Dox-inducible GenEPi mESC colony without dox treatment. (c) Dox-induced GenEPi mESC colony before and after addition of Yoda1. Scale bar, 10 µm.
- Fig. 13: shows cardiomyocyte differentiation of doxycycline-inducible GenEPi mESC cell line. (a) Time course for cardiomyocyte differentiation, leading to patches of beating cardiomyocytes. (b) Beating cardiomyocyte patches visualized in brightfield. Boxes show areas where the movement of the patch could be identified (c) The region of interest to extract the signal intensities in Figure 3. Scale bar, 5 µm.

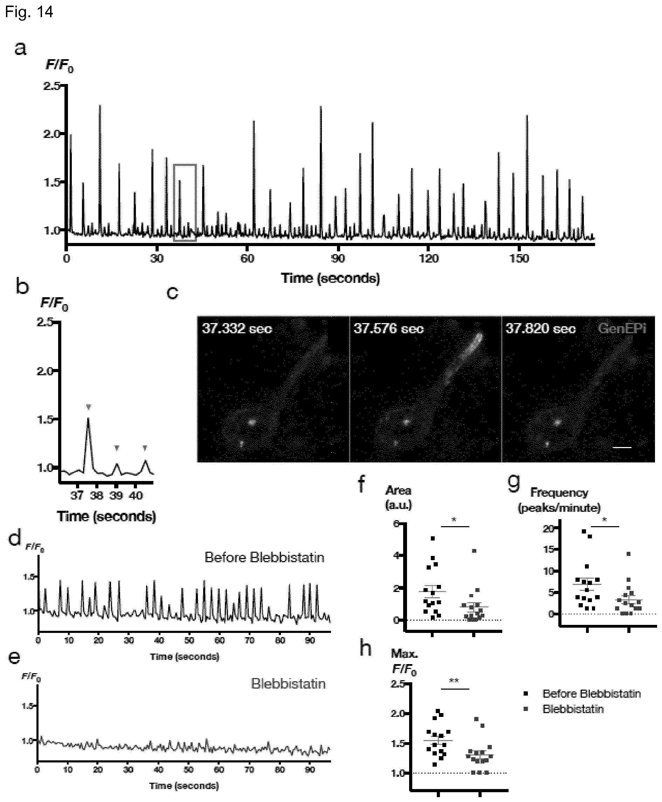

- Fig. 14: shows that GenEPi reports cardiomyocyte contraction-triggered mechanical stimulation with high spatiotemporal resolution. (a) Intensity profile of a single cell attached to the beating patch expressing GenEPi (black) in response to the autonomous subsecond beating rate of the cardiomyocytes (grey). (b) Magnified intensity profile from the boxed region in maroon in (a) of GenEPi's responses upon cardiomyocyte contraction. (c) Time-lapse images of GenEPi's responses depicted in (b). Scale bar, 5 µm.

### Examples

### Example 1: Design and results of the screen to identify a reporter for mechanical stimuli.

In a systematic screen, the inventors generated a library of reporters by fusing five different low-affinity GCaMPs (here denoted as GCaMPs-G1 - GCaMPs-G5) to the C-terminus of human Piezo1 (Fig. 1a). Given the influence of linker length on the sensing mechanism, the inventors employed flexible linker peptides with varying lengths to attach GCaMPs to Piezo1 (Fig.2a). The generated variants were evaluated based on their response to both mechanical stimuli and cytosolic Ca²⁺ fluctuations that were independent of Piezo1 activity. To test their responses to mechanical stimuli, variants were exposed to physiological levels of fluid shear stress, which causes Piezo1-dependent Ca²⁺ increase in HEK 293T cells. To test the sensitivity of the variants to intracellular Ca²⁺ levels independent of Piezo1 function, the inventors recorded their response to the Ca²⁺ ionophore ionomycin (Fig. 2b).

Among the candidates tested, the inventors identified one GCaMP-Piezo1 fusion variant, Piezo1-1xGSGG-GCaMP-G4, hereby referred to as GenEPi (Fig. 1b), whose plasma membrane and endoplasmic reticulum localization reflected proper fusion protein folding and stability (Fig. 3a, b).

### Example 2: Endoplasmic reticulum (ER) and plasma membrane localization of GenEPi and validation of whole cell fluorescence measurement

Endogenous Piezo1 localizes to the plasma membrane as well as to the ER. Moreover, when fused to fluorescent proteins, membrane ion channels can show poor membrane localization thereby reducing the resolution of the reporter signal. Therefore, the inventors investigated whether the fusion of GCaMPs to Piezo1 would affect its cellular localization. First, the inventors confirmed membrane localization of GenEPi by introducing a live membrane stain, Wheat Germ Agglutinin-conjugated Alexa Fluor 594 (magenta), to GenEPi-transfected HEK 293T cells (green). The fluorescent signal of GenEPi overlapped with the live membrane stain, indicating correct plasma membrane localization for GenEPi (Fig.4a). GCaMP-G4 alone however was excluded from the cell membrane (Fig. 4b).

Next, the inventors investigated whether the GenEPi is localized in the ER. GenEPi (green) expressing HEK293T cells were labeled with BODIPY TR Gilbenclamide (magenta), a live stain for ER. Intracellular GenEPi signal overlapped with the BODIPY TR Gilbenclamide staining, confirming ER localization of the reporter (Fig. 4c).

To confirm that the whole cell quantification of GenEPi fluorescence for various experiments reflect the changes in the membrane GenEPi signals with little interference from the ER, the inventors compared membrane signals to whole cell signals in a subset of cells. In order to isolate membrane signals, the inventors again stained GenEPi-expressing cells with WGA-Alexa Fluor 594, a live cell stain for the cell membrane (magenta). The inventors exposed the cells to 10 dyne/cm² fluid shear stress and recorded the response of GenEPi simultaneously with the static membrane staining. The inventors used the WGA-Alexa Fluor 594 channel to generate a mask of the cell membrane (Fig. 4d). This mask was applied to GenEPi signals, to extract the cell membrane-specific fluorescence signal of GenEPi. The inventors compared the responses from the membrane to that of the whole cell response of the same cells and found no significant difference between the whole cell and membrane-specific signals (Fig. 4e). Hence, whole cell responses accurately reflect cell membrane GenEPi dynamics.

### Example 3: Level or duration of applied fluid shear stress does not affect the response amplitude of GenEpi

The optical response of GenEPi to fluid shear stress (Fig. 1c) is considerably higher (1.61 ±0.098, mean ± s.e.m, n=12 cells) than that of its cytosolic counterpart (1.36 ±0.028, mean ± s.e.m, n=13 cells) (Fig. 1b), indicating that channel tethering of GCaMP-G4 in this particular configuration provides better access to high Ca²⁺ levels upon Piezo1 channel opening in response to mechanical stimuli. Furthermore, as GenEPi retained the low affinity for Ca²⁺ (Fig. 5a, b), it had a low level of response to cytosolic Ca²⁺ induced by ionomycin (1.16 ±0.054, mean ± s.e.m, n=15 cells), indistinguishable from the response levels of the control fusion protein, Piezo1-eGFP (1.12 ±0.022, mean ± s.e.m, n=19 cells) (Fig. 1b). Interestingly, changing the level (1-30 dyn/cm²) or duration (10-120 sec) of fluid shear stress did not result in any significant difference in GenEPi's response (Fig.6a, b), suggesting that its response to shear stress could be described as an all-or-nothing response. The functional specificity of GenEPi was validated by its selective response to the Piezo1-specific small molecule agonist Yoda1, which induced high levels of reporter response compared to DMSO (Fig. 1d).

### Example 4: GenEPi's response to physiological Ca²⁺ signalling

As ionomycin triggers high levels of Ca²⁺ associated with cell toxicity and death, the inventors further tested GenEPi's response to physiological Ca²⁺ signalling in the cell upon addition of 30 µM ATP (Fig. 1e). The inventors detected ATP-dependent cytosolic Ca²⁺ increase using the calcium indicator jRCaMP1a (Fig. 7a), which has no spectral crosstalk with the co-expressed GenEPi. The inventors found that the elevated Ca²⁺ levels were detected by jRCaMP1a, however, not by GenEPi (Fig. 7b). These results indicate that GenEPi is indeed responding specifically to Piezo1-dependent activity and does not sense physiological fluctuations of cytosolic Ca²⁺.

### Example 5: Plasma membrane localization of GCaMP-G4 does not confer functional specificity.

The specificity of GenEPi's response was further corroborated by the observation that membrane localization of GCaMP-G4 was not sufficient to confer functional specificity. As GenEPi responded specifically to mechanical signals and was unresponsive to cytosolic calcium fluctuations, the inventors wondered whether the demonstrated functional specificity could be also achieved by simply targeting GCaMP-G4 to the plasma membrane. To this end, the inventors attached the membrane targeting sequence of the protein tyrosine kinase Lck to the N-terminus of the calcium indicator GCaMP-G4 (Lck-GCaMP-G4). Then, the inventors exposed Lck-GCaMP-G4-transfected cells to fluid shear stress and ionomycin, and compared the responses of Lck-GCaMP-G4 and GenEPi. If simply being located to the membrane was sufficient to give i) a strong response to fluid shear stress and ii) a negligible response to ionomycin, Lck-GCaMP-G4 would be expected to behave similarly to GenEPi. In contrast, the response of Lck-GCaMP-G4 to fluid shear stress was significantly lower than that of GenEPi, while it showed a pronounced response to ionomycin (Fig. 8). This observation indicates that the calcium indicator access to cytosolic Ca²⁺ fluctuations is different when targeted to the plasma membrane compared to when it is directly attached to Piezo1.

Hence, membrane localization alone is not sufficient to acquire functional specificity to mechanical stimuli. Discrete high Ca²⁺ concentrations in the immediate vicinity of the Piezo1 channel opening could be causing these differences, such as the previously reported Ca²⁺ microdomains². Indeed, the fact that all variants gave lower responses to ionomycin (Fig. 2b) corroborates this hypothesis. Furthermore, channel tethering of all investigated GCaMP versions consistently reduced their response to cytosolic Ca²⁺ evoked by ionomycin (Fig. 2b), which suggests that genetically-encoded calcium indicators placed near the pore are protected from cytosolic Ca²⁺ fluctuations, supporting the microdomain hypothesis. Taken together, GenEPi manifests high SNR and functional selectivity to fluid shear stress.

### Example 6: GenEPi's force sensitivity and temporal resolution in response to compression

As Piezo1 is known to respond to other forms of mechanical stimuli, such as compression, the inventors characterized the force sensitivity and temporal kinetics of GenEPi under this stimulus. The inventors turned to a previously described experimental setup that allows AFM-based probing of mechanical sensitivity while simultaneously recording the optical response of GenEPi (Fig. 9a). The inventors applied fine-tuned and precisely-timed compressive forces on single HEK 293T cells expressing GenEPi using a 5µm bead attached to an AFM cantilever (Fig. 9a, b). GenEPi responded to short (250 ms) compressive forces with faster kinetics than shear stress, but on average with comparable signal amplitude (1.65, ±0.120, mean ±s.e.m, n=21 cells) (Fig. 9c). In contrast, the Piezo1-eGFP fusion did not show any optical response (Fig. 9c). The precise control of stimulation level and duration in this experimental setup allowed to characterize the force sensitivity and duration of Piezo1-induced fluorescent signals. The threshold forces measured for GenEPi transfected cells and control cells co-transfected with human Piezo1 and cytosolic GCaMP-G4 were comparable (243.5 nN, ±13.680 and 241.2 nN, ±13.870, mean ± s.e.m, each n=21 cells, respectively) (Fig. 9d), demonstrating that the force sensitivity of the channel is not affected by the protein fusion. GenEPi's response to cantilever-triggered compression lasted on average 7.569 ±1.091 seconds (mean ± s.e.m, n=16 cells), which was much shorter than that of the cytosolic indicator (18.39 ±1.841 seconds, mean ± s.e.m, n=27 cells) (Fig. 9e). Using this method, the inventors could also precisely quantify the effect of Piezo1 overexpression in conferring mechanical sensitivity to HEK 293T cells. Stimulated HEK293T cells transfected only with cytosolic GCaMP-G4 rarely responded to compressive forces (Fig. 10a); the threshold force values were considerably higher and the peak duration was substantially longer than for cells with Piezo1 overexpression (Fig. 10b, d). In conclusion, GenEPi provides not only high spatial resolution and functional specificity, but also offers a gain in temporal resolution.

### Example 7: GenEPi's response to repeated stimuli depends on the type of stimulus

GenEPi has higher temporal resolution than cytosolic GCaMP-G4. This property is advantageous when one needs to be able to resolve rapid successive optical responses to repetitive mechanical stimuli. Indeed, increasing the level of short compressive stimuli during force-spectroscopy experiments occasionally resulted in graded reporter response, where the timing and amplitude of signals could be resolved (Fig.11a, b). This observation suggests that GenEPi can report on graded responses. These results prompted us to investigate whether increasing levels of shear stress would be also captured by GenEPi. Therefore, the inventors exposed GenEPi expressing HEK293T cells to increasing levels of shear stress (5-20 dyne/cm2, each step for 30 seconds). In contrast to compressive forces, continuously increasing fluid shear stress stimulation did not produce a graded GenEPi response (Fig. 11c).

What accounts for the differences in reporting graded stimuli? Obviously, the two types of stimuli are differently exerted to and perceived by cells: whereas entire cells are under mechanical strain during fluid shear stress experiments, compressive forces are applied locally. This difference might result in a successive opening of channels with repeated application of locally increasing compressive forces, resulting in the observed consecutive fluorescent response peaks. Differences between both the duration and recovery time of these two distinct types of stimuli might also explain the differing outputs of the reporter. It has been recently proposed that the amplitude of the mouse Piezo1 response to repetitive compressive stimuli is dependent on the frequency of the applied stimulus, with more robust initial responses in higher frequency stimulations. In the experimental setup, compressive stimuli were applied for 250 milliseconds, followed by a 10 seconds recovery period, whereas the shortest time to introduce fluid shear stress was 10 seconds (Fig. 6b). Given that the opening duration of the Piezo1 channel is in the range of milliseconds, the signal of stimulated cells might have already reached saturation during fluid shear stress experiments lasting at least 10 seconds. A more precise control of fluid shear stress stimulation could potentially stimulate the reporter repeatedly and could even optically resolve differences in fluid shear stress levels. Indeed, precise control of fluid shear stress duration in astrocytes (pulse duration from 4 to 10000 milliseconds) could produce differences in the amount of intracellular Ca2+, although this change was partially contributed by intracellular Ca2+ stores. Hence, GenEPi is capable of reporting different responses of Piezo1 function to varying type and parameters of mechanical stimuli Therefore, we anticipate that GenEPi's high specificity of Piezo1 activity with its high temporal resolution will help advance the understanding of channel sensitivity and kinetics in a non-invasive manner.

### Example 8: Generation of doxvcvcline-inducible GenEPi mESC line and its use in cardiomyocyte contraction-triggered mechanical stimulation

In order to evaluate whether homeostatic cell motions, such as cardiomyocyte contraction, trigger Piezo1 activation, the inventors generated doxycycline-inducible GenEPi mouse embryonic stem cells (mESCs) (Fig. 12a) and differentiated these cells to cardiomyocytes. The inventors confirmed GenEPi's activity in undifferentiated mESC by monitoring its specific response to Yoda1 (Fig. 12b, c). At the end of the differentiation protocol, spontaneously beating patches of cells could be identified consisting of cardiomyocytes and potential other mesodermal lineage cells, such as endothelial cell and smooth muscle cells (Fig. 13). While the cardiomyocytes themselves did not produce any increase of GenEPi signal upon contraction, the inventors identified another cell connected to the beating patch that displayed robust GenEPi responses to the cardiomyocyte contraction-triggered mechanical stimulation. The signal amplitude range (1.15 to 2.29) was comparable to that of shear stress and compressive forces, yet the responses lasted less than a second (Fig. 14a-c). Interestingly, GenEPi's response was restricted to a subregion of the cell (Fig. 13c) and its frequency was slower than, but coupled to the autonomous subsecond beating rate of the cardiomyocytes. Hence, GenEPi shows a high spatiotemporal resolution of Piezo1 activity capable of noninvasively sensing repetitive mechanical stimuli of beating cardiomyocytes.

Treatment with blebbistatin, an inhibitor of myosin, leads to a decrease in signal amplitude and frequency (Fig. 14d-h). Thus, the recorded signal is specifically caused by myosin contraction.

### Example 9: Materials and methods

### Molecular cloning

The inventors obtained the human Piezo1 cDNA from Kazusa Inc, Japan. Generation of the first four types of GCaMPs; mGCaMP 6s-EF4 (GCaMP-G1), mGCaMP 6f-EF4 (GCaMP-G2), mGCaMP 6s RS1-EF3 (GCaMP-G3) and mGCaMP 6s RS1-EF4 (GCaMP-G4) are described in Helassa et al, Sci Rep 6, 38276 (2016). Fast-GCaMP-EF20 (here denoted as GCaMP-G5) was a gift from Samuel Wang (Addgene plasmid #52645). The plasmid pGP-CMV-NES-jRCaMP1a was a gift from Douglas Kim (Addgene plasmid #61462).

Piezo1 was amplified using Herculase II fusion DNA polymerase (600675, Agilent Technologies) and all calcium indicators with various linker lengths were amplified with Phusion high-fidelity DNA polymerase (M0530S, NEB). List of oligonucleotides ordered from Sigma-Aldrich are identified as SEQ ID Nos 16-27.

Piezo1 and the calcium indicators were introduced using restriction cloning. The Lck targeting sequence flanking restriction sites were synthesized by Genewiz, and introduced upstream of GCaMP-G4 and GCaMP-G5. All restriction enzymes were purchased from NEB. PCR and digestion products were purified using QIAquick PCR purification kit (28104, Qiagen) and QIAquick Gel Extraction kit (28704, Qiagen). Ligations were carried out using T4 Ligase (NEB) at 24°C for 1 hour followed by chemical transformation using Turbo ultracompetent E.coli based on K12 strain (NEB) and grown on Agar LB plates (Q60120 and Q61020, Thermo Fisher) and LB liquid media (244610, BD Bioscience) supplemented with appropriate antibiotics (100 µg/ml Ampicillin or 50 µg/ml Kanamycin, Sigma-Aldrich). Clones were screened using restriction digest and sequenced by Microsynth. Plasmid DNA isolation was carried out using ZR Plasmid Miniprep (D4054, Zymo Research).

### Cell culture

HEK293T cells were obtained from ATCC (ATCC CRL-3216). Cells were cultured at 37°C, 5% CO2, in high glucose DMEM with GlutaMAX (10569010, Thermo Fisher), supplemented with 10% FBS (P40-37500, Pan Biotech) and 1X Penicillin-Streptomycin solution (15140122, Thermo Fisher). Cells were routinely tested and were negative for mycoplasma infection using Mycoplasma detection kit (B39032, LuBioScience GmBH). Plasmid DNA for transfection was isolated from 50 ml LB culture (244610, BD Bioscience) containing appropriate antibiotics using the Zymopure Plasmid Midiprep kit (D4200, Zymo Research). The amount of DNA was measured using the Nanodrop 2000c Spectrophotometer (Thermo Fisher) and 400-800 ng of each plasmid was introduced into cells using nucleofection (V4XC-2024, Lonza). Briefly, 70-80% confluent cells were washed with 1X Dulbecco's Phosphate Buffered Saline (DPBS) (D8537-500ML, Sigma-Aldrich) and dissociated using 0.05% Trypsin-EDTA (25300054, Thermo Fisher). 10 µl of the cell suspension was mixed with 10 µl Trypan Blue (0.4%) (15250061, Thermo Fisher) and added to the cell counting slide (1450011, Bio Rad Laboratories AG). Cell count and cell viability were automatically calculated by the TC10 Cell Counter (Bio Rad Laboratories AG) and only cell suspensions with >90% viability were used for transfections. For fluid shear stress and chemical treatment experiments, 106 cells were centrifuged for 5 minutes at 90xg and resuspended in 100 µl of SF cell line nucleofector solution. The cells were then transferred into the nucleofection cuvette and pulsed using the program CM-150. 500 µl of media was added to the cells, which were then transferred to a well in a 6-well plate (140675, Thermo Fisher). At 24 hours post transfection, the cells were dissociated and counted as previously described. The cells were then seeded onto ibiTreat flow chambers (Ibidi u-slide-VI 0.4, 80606, Ibidi GmbH) for fluid shear stress experiments or ibitreat coated 8-well slides (80826, Ibidi GmbH) with a density of 75,000 cells per channel or well.

For experiments to test the response of the reporter to various chemicals, the inventors used 1 µM ionomycin (I3909-1ML, Sigma-Aldrich), 30 µM ATP (A6559-25UMO, Sigma Aldrich) diluted in DPBS or 10 µM Yoda1 (5586, Tocris Bioscience) diluted in DMSO (D8418, Sigma Aldrich).

For AFM experiments, HEK 293T cells were transfected using lipofection. Briefly, 1.5 106 cells were seeded in a T25 flask (CLS430639, Sigma-Aldrich) the day before transfection. 4 µg of the reporter, or 1.6 µg of GCaMP-G4 and 3.6 µg of Piezo1 were diluted in 250 µl of Opti-MEM (31985062, Thermo Fisher), while 20 µl of Lipofectamine 2000 reagent (11668019, Thermo Fisher) was also diluted in 250 µl of Opti-MEM. After 5 minutes of incubation, the two solutions were mixed together and further incubated for 20 minutes. The solution was then introduced to cells and washed away with fresh medium after 4 hours. At 24 hours post transfection, the cells were dissociated and counted as previously described, and seeded onto 35 mm-wide cover-glass bottom Fluorodishes (FD35-100, World Precision Instruments), with a density of 300,000 cells per plate.

For the in situ affinity measurements, HeLa cells were cultured in DMEM containing non-essential amino-acids (Life Technologies), penicillin/streptomycin (100 U/ml, 100 µg/ml, respectively) and 10% heat inactivated FBS (Life Technologies) at 37 °C in an atmosphere of 5% CO2. Cells were plated on 35 mm glass bottom culture dishes (MatTek) and allowed 24 hours to adhere before transfection with FuGENE HD (Promega) following the manufacturer's instructions. Cells were maintained for 12-24 hours before being used in experiments.

Doxycycline-inducible GenEPi-mESCs were generated using ZX1 mESCs carrying rtTA in the Rosa26 locus and dox-inducible cre flanked by self-incompatible LoxP sites in the HPRT locus4, kindly provided by Dr. Michael Kyba. ZX1 mESCs were cultured in DMEM (Life Technologies), 15% FBS (PAN Biotech), 2 mM L-Glutamine (Invitrogen), 1X non-essential amino acids, 0.1 mM β-mercaptoethanol, 100U/mL leukemia inhibitory factor (Peprotech), 1 µM PD0325901 (Selleckchem) and 3 µM CHIR99201 (R&D Systems) on gelatin coated plates. Prior to electroporation, ZX1 mESCs were exposed to 500 nl/mL doxycycline for 24 hours. 1 x 106 ZX1 mESCs were electroporated with 3 µg p2lox plasmid in which GenEPi was cloned between LoxP sites in a 0.4 cm electroporation cuvette at 230 mV, 500 µF and maximum resistance in a Biorad electroporator (Biorad Genepulser Xcell). 24 hours after electroporation, antibiotic selection was started with 300 µg/mL G418 (Sigma). Colonies that incorporated GenEPi were verified by FACS analysis and expanded. Dox-inducible GenEPi mESCs were differentiated to cardiomyocytes as previously described⁵. GenEPi mESCs were seeded as 500 cell / 20 µl in hanging drops on non-adherent plates to generate embryoid bodies (EBs) in EB medium, IMDM (Life Technologies), 20% FBS (PAN Biotech), 2 mM L-Glutamine (Invitrogen), 1X non-essential amino acids and 0.1 mM β-mercaptoethanol. After 2 days, EBs were transferred to uncoated petridishes. From day 3-5 1 µM XAV939 was added to the culture conditions and EBs were plated on gelatine coated dished from d4. Beating EBs appeared at day 10 of differentiation. Beating EBs were manually dissected and dissociated using 2 mg/ml Collagenase/Dispase (Sigma) to generate smaller beating patches and single cells.

### Fluid shear stress applications

The inventors used the ibidi pump system (#10905, Ibidi GmBH). Fluid shear stress levels were calibrated and imaging solution viscosity of the perfusion solution was determined according to manufacturer's instructions. Depending on the level of fluid shear stress applied, perfusion set yellow-green (#10964, for 5-30 dyne/cm2) or perfusion set white (#10963, for 1-5 dyne/cm2) were used. Representative fluid shear stress application traces are shown in Fig. 1c.

### Confocal microscopy

Images were acquired using the Zeiss 780 NLO Confocal 3 equipped with an argon laser for 458 and 488 nm excitation, a diode pumped solid-state laser for 561 nm excitation and a HeNe laser for 633 nm excitation. Single cells were acquired using the C Apo 40x/1.1 W DICIII objective, excited with 488 nm for reporter, and 561 nm for tdTomato and jRCaMP1a excitation, respectively. In order to ensure fast image acquisition, the inventors imaged single cells in a small region of interest within the field of view, recording a single z-plane over several minutes. Live imaging of cells was carried out in Live Cell Imaging Solution (A14291DJ, ThermoFisher).

### Atomic Force Microscopy (AFM)-based force spectroscopy and simultaneous confocal microscopy

Prior to the experiment, 5 µm diameter silica beads (Kisker Biotech) were glued to the free end of tipples cantilevers (CSC-37, Micromash HQ) using UV glue (Dymax) and cured under UV light for 20 minutes. Cantilevers with beads were plasma treated for 5 minutes using plasma cleaner (Harrick Plasma) to ensure a clean surface, and subsequently mounted on a standard glass cantilever holder (JPK Instruments) of the AFM. Cells cultured on glass-bottom Petri dishes were kept at 37°C using a Petri dish heater (JPK Instruments). For the mechanical stimulation, an AFM (CellHesion 200, JPK Instruments) was mounted on an inverted confocal microscope (Observer Z1, Zeiss LSM 700). Cantilevers were calibrated using the thermal noise method (Hutte. & Bechhoefer, Review of Scientific Instruments 64, 1868-1873, 1998). Mechanical stimulation protocols were programmed using the JPK CellHesion software. During the mechanical stimulus, the AFM lowered the bead on the cantilever onto the cell with a speed of 10 µm s⁻¹ until the force reached the set point, held the set point force at constant force for 250 milliseconds, and then retracted with a speed of 100 µm s⁻¹. Set point forces were applied in intervals from 100 nN to 400 nN with 50 nN increments and time between intervals ranged from 10-25 seconds. Representative mechanical stimulation traces are shown in Fig. 9d.

Confocal imaging was performed using an inverted laser-scanning microscope (Zeiss LSM 700) equipped with a 25x/0.8 LCI PlanApo water immersion objectiv. Time-lapse images were acquired with 100-300 milliseconds time resolution and acquisition was initiated > 10 seconds before the onset of the mechanical stimulus. Time-lapse images of calcium responses were analysed using the built in ZEN blue software.

### In situ Ca2+ titration of GenEPi

GenEPi-transfected HeLa cells were permeabilized using 150 µM β-escin (in 20 mM Na+-HEPES, 140 mM KCI, 10 mM NaCl, 1 mM MgCI2, pH 7.2) for 4 minutes. The solution was replaced with "zero free Ca2+" solution (20 mM Na+-HEPES, 140 mM KCI, 10 mM NaCl, 1 mM MgCI2, 10 mM EGTA, pH 7.2) and various Ca2+ concentrations (0.001, 0.01, 0.1, 1, 10, 50, 500, 10000 µM free Ca2+) were applied in the presence of 10 µM ionomycin and 4 µM thapsigargin. Free Ca2+ concentrations were calculated using the two-chelators Maxchelator program7.

Cells were examined with a Zeiss LSM 800 confocal microscope equipped with a 63x/1.4 Plan-Apochromat oil immersion objective and a 488 nm diode laser as excitation light source. Emitted light was collected through Variable Secondary Dichroics (VSDs) onto a GaAsP-PMT detector. The fluorescence signal was monitored over an elliptical region of interest (ROI) in the plasma membrane using the ImageJ program. Data obtained from 12 to 42 cells (from at least three independent experiments) was plotted and analysed on GraphPad Prism 6. The fluorescence dynamic range (Fmax-FO)/FO or ΔF/F0 was expressed as mean ± s.e.m. The Ca2+ dissociation constant (Kd) and cooperativity (n) were obtained by fitting the data to the Hill equation.

### Live staining and immunochemistry

Live cell staining of cells was achieved using the Image-IT LIVE plasma membrane and nuclear labelling kit cell staining kit (134406, Thermo-Fisher), and the ER tracker red (E34250, Thermo-Fisher) according to product specifications. For antibody staining, cells were fixed in 4% paraformaldehyde (15714-S, Lucerna Chem AG) for 5 minutes, washed with PBS and blocked using Max Block blocking medium (15252, Active Motif) supplemented with 0.1% TritonX-100 (T8787, Sigma Aldrich). Cells were then incubated with anti-GFP antibody (ab6673, Abcam) or anti-Piezol antibody (ab82336, Abcam) diluted in Max Block blocking medium. After several washing steps with PBS, the cells were incubated with goat anti-rabbit Alexa Fluor-633 (A-21071) or donkey anti-goat Alexa Fluor-633 (A-21082, Thermo Fisher) as well as DAPI (62248, Thermo Fisher).

### Image processing and analysis

For the shear stress experiments, and time-lapse upon experiments with application of a chemical, the cells were automatically segmented using a MATLAB script. Briefly, the signals from the cytosolic tdTomato or jRCaMP1a were automatically identified, and high intensity pixels were used to generate a mask. This mask was then applied to the time series images of the reporter and the cytosolic signal and single cell intensities were extracted for each time point. This information allowed us to get the traces for the intensiometric reporter response of each cell, changing fluorescence (F) divided by the first five frames to get a baseline fluorescence of the cell (F0), expressed as change in fluorescence over time (F/F0).

During the AFM experiments, mechanical stimulation of cells with the cantilever caused cytosolic or membrane bound fluorophores to move in or out of the confocal imaging plane, creating fluorescence artefacts. These artifacts were clearly distinguishable from Piezo1 receptor mediated calcium influx, since (i) they showed strong symmetry with stepwise increase and decrease of fluorescence (ii) were very short in duration, and (iii) appeared synchronously with the mechanical stimulus and were thus preceding the calcium responses. Fig. 2f illustrates such an example of fluorescence artifacts and calcium responses. Any fluorescent signal that was greater than the artifact was classified as "response", anything below was defined as noise. The resulting signal trace was processed as described above. The duration of the signals were calculated by substracting the first time point fluorescence signal is higher than artifact from the time point signal goes back to the baseline. Baseline was calculated as average fluorescence of 5 seconds preceding the stimulus.

### Statistical analysis

All data are expressed means ±s.e.m. Sample sizes (n) are provided in the text or figure legend of each experiment. Each experiment has been repeated independently at least 3 times. Each data set was subjected to Shapiro-Wilk normality test to determine whether the data set has a Gaussian distribution; P>0.05 indicated it has a Gaussian distribution, and P<0.05 indicated it did not. When all of the compared data sets had Gaussian distribution, two-tailed Student's t-test was applied to compare two datasets; with an F-test to compare variances. When F-test resulted in P<0.05, Welch's correction was applied to the t-test. When more than 2 datasets were present with Gaussian distribution, one-way ANOVA was used to compare datasets, followed by Holm-Sidak's post hoc multiple comparisons test. When at least one of the compared data sets did not have a Gaussian distribution, Mann-Whitney test was applied to compare two datasets. When more than 2 datasets were present without Gaussian distribution, Kruskal-Wallis test was applied followed by Dunn's post hoc multiple comparisons test. For all statistics, P value was reported, with n.s.=P>0.05, *=P<0.05,**= P<0.01 and ***=P<0.001.

### Sequences

SEQ ID 01: (nucleic acid; homo sapiens; PIEZO 1)
SEQ ID 02: (nucleic acid; artificial sequence; circularly permuted GFP)
SEQ ID 03: (nucleic acid; artificial sequence; Calmodulin)
SEQ ID 04: (amino acid; artificial sequence; modulator peptide sequence 1)
SEQ ID 05: (amino acid; artificial sequence; modulator peptide sequence 2)
SEQ ID 06: (amino acid; artificial sequence; modulator peptide sequence 3)
SEQ ID 07: (amino acid; artificial sequence; modulator peptide sequence 4)
SEQ ID 08: (amino acid; artificial sequence; modulator peptide sequence 5)
SEQ ID 09: (amino acid; artificial sequence; modulator peptide sequence 6)
SEQ ID 10: (amino acid; modulator peptide sequence 7)
SEQ ID 11: (amino acid; artificial sequence; modulator peptide sequence 8)
SEQ ID 12: (amino acid; artificial sequence; modulator peptide sequence 9)
SEQ ID 13: (nucleic acid; artificial sequence; cpGFP-RS20-Calmodulin)
SEQ ID 14: (nucleic acid; artificial sequence; PIEZO 1 - cpGFP- RS20-Calmodulin)
SEQ ID 15: (nucleic acid; artificial sequence; 5' His tag, RS20, circularly permuted GFP, CaM enhancer 3')
SEQ ID 16: (nucleic acid; artificial sequence; Aqel-F (GCaMPs-G1-G5))
SEQ ID 17: (nucleic acid; artificial sequence; 1 xGSGG-F (GCaMPs-G1-G5))
SEQ ID 18: (nucleic acid; artificial sequence; 2xGSGG-F (GCaMPs-G1-G5))
SEQ ID 19: (nucleic acid; artificial sequence; GCaMP-R-Notl (GCaMPs-G2-G5))
SEQ ID 20: (nucleic acid; artificial sequence; R-6s-EF4 (GCaMP-G1))
SEQ ID 21: (nucleic acid; artificial sequence; hP1-F-HindIII)
SEQ ID 22: (nucleic acid; artificial sequence; hP1-R-Aciel)
SEQ ID 23: (nucleic acid; artificial sequence; egfp-F)
SEQ ID 24: (nucleic acid; artificial sequence; egfp-R)
SEQ ID 25: (nucleic acid; artificial sequence; hP1-F-Agel)
SEQ ID 26: (nucleic acid; artificial sequence; hP1-R-Notl)
SEQ ID 27: (nucleic acid; artificial sequence; gBlock containing Lck targeting sequence)

## Claims

1. A polypeptide comprising:
d. a first polypeptide module comprising a transmembrane pore-forming polypeptide permeable for a calcium ion, wherein the permeability of said pore-forming polypeptide is modulated by a stimulus, particularly a mechanical stimulus;
e. a second polypeptide module comprising
- a luminescent polypeptide, particularly a fluorescent polypeptide,
- a calcium binding polypeptide capable of selectively binding a calcium ion, particularly wherein the binding of said polypeptide to said calcium ion is **characterized by** a dissociation constant K_{D} in the range of 100 nmol/L to 10 µmol/L, more particular said K_{D} is in the range 500 nmol/L to 2 µmol/L, and
- a modulator peptide sequence capable of binding to said calcium binding polypeptide, wherein
- said modulator peptide sequence has a higher affinity to said calcium binding polypeptide when said calcium binding sequence is bound to said calcium ion than when said calcium binding sequence is not bound to said calcium ion,
- and wherein luminescence of said luminescent polypeptide alters upon binding of said modulator peptide sequence to said calcium binding polypeptide
f. a peptide linker sequence covalently connecting said first polypeptide module and said second polypeptide module.

2. The polypeptide according to claim 1, wherein said transmembrane pore-forming polypeptide permeable for a calcium ion comprises or consists of the amino acid sequence of SEQ ID 01 or a functional equivalent thereof being ≥85%, 90%, 92%, 94%, 96%, particularly 98% or 99%, identical to SEQ ID 01.

3. The polypeptide according to any one of preceding claims, wherein said luminescent polypeptide is a fluorescent polypeptide selected from
(i) a green fluorescent polypeptide, particularly an enhanced green fluorescent polypeptide, more particularly a circularly permuted green fluorescent polypeptide;
(ii) a red fluorescent polypeptide, particularly an enhanced red fluorescent polypeptide;
(iii) a blue fluorescent polypeptide, particularly an enhanced blue fluorescent polypeptide;
(iv) a yellow fluorescent polypeptide, particularly an enhanced yellow fluorescent polypeptide;
(v) a cyan fluorescent polypeptide, particularly an enhanced cyan fluorescent polypeptide.

4. The polypeptide according to any one of preceding claims, wherein said calcium binding polypeptide comprises or consists of an amino acid sequence encoded by a nucleic acid sequence **characterized by** SEQ ID 03 or a functional equivalent thereof being ≥85%, 90%, 92%, 94%, 96%, particularly 98% or 99%, identical to said amino acid sequence encoded by a nucleic acid sequence **characterized by** SEQ ID 03.

5. The polypeptide according to any one of preceding claims, wherein said modulator peptide sequence has an amino acid length of 5 to 30 amino acids, particularly a length of 10 to 25 amino acids and wherein said modulator peptide sequence is capable of forming a hydrophobic interaction with said calcium binding polypeptide.

6. The polypeptide according to any one of preceding claims, wherein said modulator peptide sequence comprises or consists of the amino acid sequence selected from SEQ ID 04, SEQ ID 05, SEQ ID 06, SEQ ID 07, SEQ ID 08, SEQ ID 09, SEQ ID 10, SEQ ID 11, SEQ ID 12, or a functional equivalent thereof being ≥85%, 90%, 92%, 94%, 96%, particularly 98% or 99%, identical to SEQ ID 04.

7. The polypeptide according to any one of preceding claims, wherein said second polypeptide module comprises or consists of an amino acid sequence encoded by a nucleic acid sequence **characterized by** SEQ ID 13 or a functional equivalent thereof being ≥85%, 90%, 92%, 94%, 96%, particularly 98% or 99%, identical to said amino acid sequence encoded by a nucleic acid sequence **characterized by** SEQ ID 13.

8. The polypeptide according to any one of preceding claims, wherein said polypeptide comprises or consists of an amino acid sequence encoded by a nucleic acid sequence **characterized by** of SEQ ID 14 or a functional equivalent being ≥85%, 90%, 92%, 94%, 96%, particularly 98% or 99%, identical to said amino acid sequence encoded by a nucleic acid sequence **characterized by** SEQ ID 14.

9. The polypeptide according to any one of preceding claims, wherein said peptide linker sequence is **characterized by**
(iii) a length in the range of 1 to 10 amino acids residues, particularly 2 to 8 amino acids residues, more particular 4 amino acids residues, and/or
(iv) an amino acid sequence comprising glycine and serine residues, particularly an amino acid sequence of Gly-Ser-Gly-Gly.

10. A polynucleotide sequence encoding the polypeptide according to any one of preceding claims.

11. A method for detection of a stimulus applied to a cell, comprising the steps of:
(iii) providing a host cell comprising a polynucleotide sequence according to claim 10,
(iv) determining a fluorescence of said host cell,
wherein said fluorescence changes in dependence of said stimulus applied to said host cell.

12. The method according to claim 11, wherein said stimulus is a mechanical stimulus.

13. The method according to claims 11 and 12, further comprising the step applying a mechanical stimulus to said host cell, particularly wherein said mechanical stimulus comprises a mechanical force stimulus selected from:
(iv) shear force, particularly fluid shear force,
(v) pressure force, particularly cyclic pressure,
(vi) stretch force, particularly cyclic stretch.

14. The method according to claims 11 to 13, wherein said fluorescence is measured by an excitation at 488nm and a change of measured fluorescence is detected after applying said mechanical stimulus in a range of 0 to 400nN or in a range of 0-20 dyne/cm² over a time period of 100 to 200 msec.

15. A method to assess the ability of a test compound to modulate the permeability of transmembrane pore-forming polypeptide permeable for a calcium ion, wherein said transmembrane pore-forming polypeptide permeable for a calcium ion is comprised in a polypeptide according to any one of claims 1 to 9, and a method according to any one of claims 11 to 14 is performed in presence and absence of said test compound, and modulation of said transmembrane pore-forming polypeptide is assessed by comparison of results obtained by said method in presence and absence of said test compound.
